(19) Europäisches Patentamt
European Patent Office
Office européen des brevets

(11) EP 1 216 996 A2

(12) # DEMANDE DE BREVET EUROPEEN

(43) Date de publication:
**26.06.2002 Bulletin 2002/26**

(51) Int Cl.[7]: **C07D 211/70**, C07D 401/04

(21) Numéro de dépôt: **02006482.0**

(22) Date de dépôt: **26.06.1996**

(84) Etats contractants désignés:
**AT BE CH DE DK ES FI FR GB GR IE IT LI LU MC NL PT SE**
Etats d'extension désignés:
**LT LV SI**

(30) Priorité: **28.06.1995 FR 9507760**

(62) Numéro(s) de document de la (des) demande(s) initiale(s) en application de l'article 76 CBE:
**96924023.3 / 0 837 848**

(71) Demandeur: **SANOFI-SYNTHELABO**
**75013 Paris (FR)**

(72) Inventeurs:
- **Badone, Domenico**
  **21056 Induno Olona (IT)**
- **Baroni, Marco**
  **20010 Vanzago (IT)**
- **Cardamone, Rosanna**
  **22100 Como (IT)**
- **Fournier, Jacqueline**
  **31830 Plaisance-du-Touch (FR)**
- **Guzzi, Umberto**
  **20148 Milano (IT)**
- **Ielmini, Alessandra**
  **21010 Arsago Seprio (IT)**

(74) Mandataire: **Nevant, Marc et al**
**Cabinet Beau de Loménie,**
**158, rue de l'Université**
**75007 Paris Cedex 07 (FR)**

Remarques:
Cette demande a été déposée le 22 - 03 - 2002 comme demande divisionnaire de la demande mentionnée sous le code INID 62.

(54) **Procédé de préparation de dérives biphenylyl**

(57) L'invention concerne un procédé pour la préparation de dérivés biphénylyl par condensation entre des dérivés phényl substitués par un groupe partant et des acides benzèneboroniques, en présence d'un catalyseur, d'une base forte et d'un agent de transfert de phase.

EP 1 216 996 A2

## Description

La présente invention concerne de nouvelles 1-phénylalkyl-1,2,3,6-tétrahydropyridines 4-substituées ayant une activité neurotrophique et neuroprotectrice, un procédé pour leur préparation, et des compositions pharmaceutiques les contenant.

EP-0 458 696 décrit l'utilisation d'une 1-(2-naphtyléthyl)-4-(3-trifluorométhylphényl)-1,2,3,6-tétrahydropyridine pour la préparation de médicaments destinés au traitement de troubles cérébraux et neuronaux.

WO 93/11107 décrit des pipéridines et tétrahydropyridines à activité protectrice vis-à-vis des dommages causés par les états hypoxiques/ischémiques.

Il a été maintenant trouvé que certaines phénylalkyl-1,2,3,6-tétrahydropyridines, substituées par un groupe phényle ou pyridyle, exercent une action neurotrophique sur le système nerveux semblable à celle du facteur de croissance nerveuse (NGF de l'anglais Nerve Growth Factor) et peuvent rétablir le fonctionnement des cellules endommagées ou présentant des anomalies dans leurs fonctions physiologiques.

La présente invention concerne donc, selon un de ses aspects, les composés de formule (I)

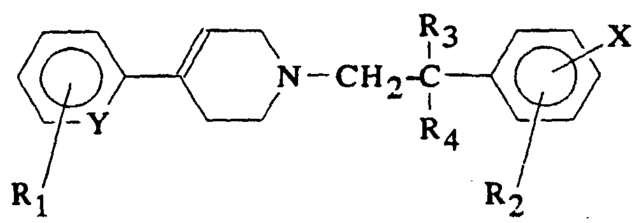

(I)

dans laquelle

- Y représente -CH- ou -N-;
- $R_1$ représente l'hydrogène, un halogène, un groupe $CF_3$, $(C_3$-$C_4)$alkyle ou $(C_1$-$C_4)$-alcoxyle;
- $R_2$ représente l'hydrogène, un halogène, un hydroxyle, un groupe $CF_3$, $(C_3$-$C_4)$-alkyle ou $(C_1$-$C_4)$alcoxyle;
- $R_3$ et $R_4$ représentent chacun l'hydrogène ou un $(C_1$-$C_3)$alkyle;
- x représente

(a) un $(C_3$-$C_6)$alkyle; un $(C_3$-$C_6)$alcoxyle; un $(C_3$-$C_7)$carboxyalkyle; un $(C_1$-$C_4)$alcoxycarbonyl$(C_3$-$C_6)$alkyle; un $(C_3$-$C_7)$carboxyalcoxyle; ou un $(C_1$-$C_4)$alcoxycarbonyl$(C_3$-$C_6)$alcoxyle;

(b) un radical choisi parmi un $(C_3$-$C_7)$cycloalkyle, $(C_3$-$C_7)$cycloalkyloxy, $(C_3$-$C_7)$cycloalkylméthyle, $(C_3$-$C_7)$cycloalkylamino et cyclohexényle, ledit radical pouvant être substitué par un halogène, hydroxy, $(C_1$-$C_4)$alcoxy, carboxy, $(C_1$-$C_4)$alcoxycarbonyle, amino, mono- ou di-$(C_1$-$C_4)$alkylamino; ou

(c) un groupe choisi parmi un phényle, phénoxy, phénylamino, N-$(C_1$-$C_3)$alkyl-phénylamino, phénylméthyle, phényléthyle, phénylcarbonyle, phénylthio, phénylsulfonyle, phénylsulfinyle ou styryle, ledit groupe pouvant être mono- ou polysubstitué sur le groupe phényle par un halogène, $CF_3$, $(C_1$-$C_4)$alkyle, $(C_1$-$C_4)$alcoxy, cyano, amino, mono- ou di-$(C_1$-$C_4)$-alkylamino, $(C_1$-$C_4)$acylamino, carboxy, $(C_1$-$C_4)$alcoxycarbonyle, aminocarbonyle, mono- ou di-$(C_1$-$C_4)$alkylaminocarbonyle, amino$(C_1$-$C_4)$alkyle, hydroxy$(C_1$-$C_4)$alkyle ou halogéno$(C_1$-$C_4)$alkyle;

ainsi que leurs sels et solvates et leurs sels d'ammonium quaternaire.

Dans la présente description le terme "$(C_1$-$C_3)$alkyle" désigne les groupes méthyle, éthyle, *n*-propyle et *i*-propyle.

Le terme "$(C_1$-$C_4)$alkyle" désigne les groupes méthyle, éthyle, n-propyle, i-propyle, n-butyle, i-butyle, s-butyle et t-butyle.

Le terme "$(C_3$-$C_6)$alkyle" désigne un radical hydrocarbonné saturé ou insaturé, contenant de 3 à 6 atomes de carbone tel que, par exemple, *n*-propyle, *i*-propyle, *n*-butyle, *i*-butyle, *s*-butyle, *t*-butyle, *n*-pentyle, *i*-pentyle, néopentyle, *t*-pentyle, *n*-hexyle, *i*-hexyle, etc.

Le terme "alcoxy" désigne un groupe hydroxyle substitué par un groupe alkyle, alcényle ou alcynyle.

Lorsque X représente un groupe phényle, la nomenclature donnée au radical biphénylique est celle conforme aux règles IUPAC, à savoir la numérotation des positions des deux cycles est la suivante:

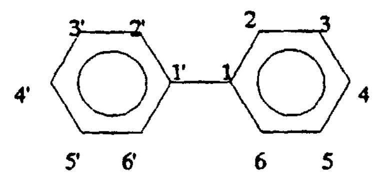

et les radicaux ayant cette structure sont nommés

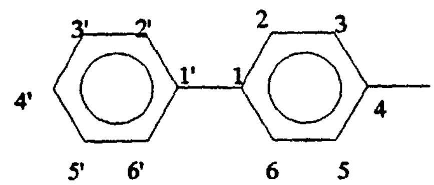

4-biphénylyl-

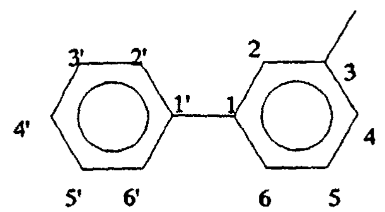

3-biphénylyl-

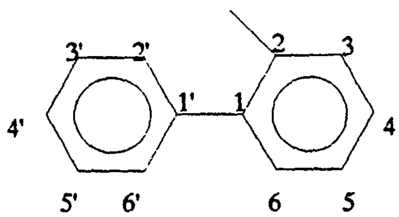

2-biphénylyl-

Les composés de formule (I) où X est dans la position 4 du groupe phényle et leurs sels, notamment ceux pharmaceutiquement acceptables, leurs solvates et leurs sels d'ammonium quaternaire, sont des composés particulièrement avantageux.

Parmi les composés de formule (I) où X est un groupe (c), un groupe préféré est représenté par les composés où le phényle est substitué par 1 à 3 halogène, 1 à 3 $CF_3$, 1 à 3 ($C_1$-$C_4$)alkyle, 1 à 3 ($C_1$-$C_4$)alcoxy, 1 à 3 cyano, 1 à 3 amino, 1 à 3 mono- ou di-($C_1$-$C_4$)alkylamino, 1 à 3 ($C_1$-$C_4$)acylamino, 1 à 3 carboxy, 1 à 3 ($C_1$-$C_4$)-alcoxycarbonyle, 1 à 3 aminocarbonyle, 1 à 3 mono- ou di-($C_1$-$C_4$)alkylaminocarbonyle, 1 à 3 amino($C_1$-$C_4$)alkyle, 1 à 3 hydroxy($C_1$-$C_4$) alkyle ou 1 à 3 halogéno($C_1$-$C_4$)alkyle.

Un autre groupe préféré est constitué par les composés de formule (I) où Y est un groupe -CH- et $R_1$ est $CF_3$.

Un autre groupe préféré est constitué par les composés de formule (I) où Y est un atome d'azote et $R_1$ est un atome de chlore.

Des composés particulièrement avantageux selon la présente invention sont les suivants :

1-[2-(4-isobutylphényl)propyl]-4-(3-trifluorométhylphényl)-1,2,3,6-tétrahydropyridine;
1-[(2S)-2-(4-isobutylphényl)propyl]-4-(3-trifluorométhylphényl)-1,2,3,6-tétrahydropyridine;
1-[(2R)-2-(4-isobutylphényl)propyl]-4-(3-trifluorométhylphényl)-1,2,3,6-tétrahydropyridine;
1-[2-(4-isobutylphényl)éthyl]-4-(3-trifluorométhylphényl)-1,2,3,6-tétrahydropyridine;
1-[2-(4-tertbutylphényl)éthyl]-4-(3-trifluorométhylphényl)-1,2,3,6-tétrahydropyridine;
1-[2-(4-isobutylphényl)-2-méthylpropyl]-4-(3-trifluorométhylphényl)-1,2,3,6-tétrahydropyridine;
1-[2-(4-isopropylphényl)éthyl]-4-(3-trifluorométhylphényl)-1,2,3,6-tétrahydropyridine;
1-[2-(3'-chloro-4-biphénylyl)éthyl]-4-(3-trifluorométhylphényl)-1,2,3,6-tétrahydropyridine;
1-[2-(2'-chloro-4-biphénylyl)éthyl]-4-(3-trifluorométhylphényl)-1,2,3,6-tétrahydropyridine;
1-[2-(4'-chloro-4-biphénylyl)éthyl]-4-(3-trifluorométhylphényl)-1,2,3,6-tétrahydropyridine;

1-[2-(4'-fluoro-4-biphénylyl)éthyl]-4-(3-trifluorométhylphényl)-1,2,3,6-tétrahydropyridine;
1-[2-(3'-trifluorométhyl-4-biphénylyl)éthyl]-4-(3-trifluorométhylphényl)-1,2,3,6-tétrahydropyridine;
1-[2-(4-cyclohexylphényl)éthyl]-4-(3-trifluorométhylphényl)-1,2,3,6-tétrahydropyridine;
1-[2-(4-biphénylyl)-2-éthyl]-4-(4-fluorophényl)-1,2,3,6-tétrahydropyridine;
1-[2-(4-biphénylyl)-2-méthylpropyl]-4-(3-trifluorométhylphényl)-1,2,3,6-tétrahydropyridine;
1-[2-(4-phénoxyphényl)-2-éthyl]-4-(3-trifluorométhylphényl)-1,2,3,6-tétrahydropyridine;
1-[2-(4-benzylphényl)-2-éthyl]-4-(3-trifluorométhylphényl)-1,2,3,6-tétrahydropyridine;
1-[2-(4-n-butylphényl)éthyl]-4-(3-trifluorométhylphényl)-1,2,3,6-tétrahydropyridine;
1-[2-(4-biphénylyl)éthyl]-4-(3-trifluorométhylphényl)-1,2,3,6-tétrahydropyridine;
1-[2-(4-n-butoxyphényl)éthyl]-4-(3-trifluorométhylphényl)-1,2,3,6-tétrahydropyridine;
1-[2-(4-(3-éthoxycarbonylpropoxy)phényl)éthyl]-4-(3-trifluorométhylphényl)-1,2,3,6-tétrahydropyridine;
1-[2-(4-biphénylyl)éthyl]-4-(6-chloropyrid-2-yl)-1,2,3,6-tétrahydropyridine;
1-[2-(2,3'-dichloro-4-biphenylyl)éthyl]-4-(3-trifluorométhylphényl)-1,2,3,6,-tétrahydropyridine;
1-[2-(3-chloro-4-biphénylyl)éthyl]-4-(3-trifluorométhylphényl)-1,2,3,6-tétrahydropyridine;
1-[2-(3',5'-dichloro-4-biphénylyl)éthyl]-4-(3-trifluorométhylphényl)-1,2,3,6-tétrahydropyridinc;
1-[2-(2',4'-dichloro-4-biphénylyl)éthyl]-4-(3-trifluorométhylphényl)-1,2,3,6-tétrahydropyridine;
1-[2-(2-chloro-4-biphénylyl)éthyl]-4-(3-trifluorométhylphényl)-1,2,3,6-tétrahydropyridine;
1-[2-(3'-chloro-4-biphénylyl)-2-méthylpropyl]-4-(3-trifluorométhylphényl)-1,2,3,6-tétrahydropyridine;
1-[2-(2-fluoro-4-biphénylyl)propyl]-4-(3-trifluorométhylphényl)-1,2,3,6-tétrahydropyridine;
1-[2-(4-méthoxy-3-biphénylyl)éthyl]-4-(3-trifluorométhylphényl)-1,2,3,6-tétrahydropyridine;
1-[2-(4'-méthoxy-4-biphénylyl)éthyl]-4-(3-trifluorométhylphényl)-1,2,3,6-tétrahydropyridine;
1-[2-(4'-hydroxy-4-biphénylyl)éthyl]-4-(3-trifluorométhylphényl)-1,2,3,6-tétrahydropyridine;
1-[2-(4'-éthoxycarbonylbutoxy-4-biphénylyl)éthyl]-4-(3-trifluorométhylphényl)-1,2,3,6-tétrahydropyridine;
1-[2-(3-biphénylyl)éthyl]-4-(3-trifluorométhylphényl)-1,2,3,6-tétrahydropyridine;
1-[2-(3'-chloro-4'-fluoro-4-biphénylyl)éthyl]-4-(3-trifluorométhylphényl)-1,2,3,6-tétrahydropyridine;
1-[2-(2'-trifluorométhyl-4-biphénylyl)éthyl]-4-(3-trifluorométhylphényl)-1,2,3,6-tétrahydropyridine;
1-[2-(3,4-diisobutylphényl)éthyl]-4-(3-trifluorométhylphényl)-1,2,3,6-tétrahydropyridine;
1-[2-(3,4-dipropylphényl)éthyl]-4-(3-trifluorométhylphényl)-1,2,3,6-tétrahydropyridine;
1-[2-(4-cyclohexylphényl)éthyl]-4-(6-chloropyrid-2-yl)-1,2,3,6-tétrahydropyridine;
1-[2-(4-isobutylphényl)propyl]-4-(6-chloropyrid-2-yl)-1,2,3,6-tétrahydropyridine;

et leurs sels.

Selon un autre de ses aspects, la présente invention concerne un procédé pour la préparation des composés de formule (I), de leurs sels ou solvates et de leurs sels d'ammonium quaternaire, caractérisé en ce que

(a) on fait réagir une aryl-1,2,3,6-tétrahydropyridine de formule (II)

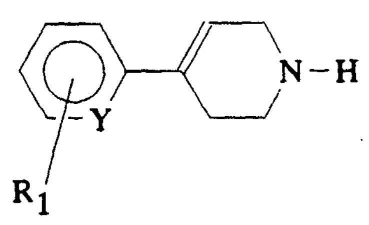

(II)

dans laquelle Y et $R_1$ sont tels que définis ci-dessus, avec un composé de formule (III)

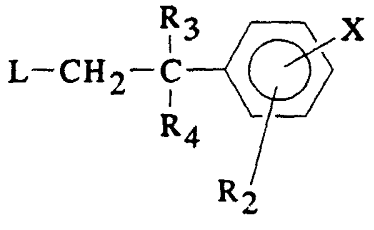

(III)

dans laquelle $R_2$, $R_3$, $R_4$ et X sont tels que définis précédemment et L représente un groupe partant, tel que par

exemple un atome de chlore, de brome, d'iode, le groupe méthanesulfonyloxy, p-toluènesulfonyloxy, trifluorométhylsulfonyloxy;

(b) on isole le composé de formule (I) ainsi obtenu et, éventuellement on le transforme en l'un de ses sels ou solvates ou l'un de ses sels d'ammonium quaternaire.

La réaction est conduite dans un solvant organique et à une température comprise entre la température ambiante et la température de reflux du solvant utilisé.

Comme solvant organique préférentiel, on utilise un alcool aliphatique ayant de 1 à 6 atomes de carbone, tel que méthanol, éthanol, isopropanol, n-butanol, n-pentanol, mais aussi d'autres solvants tels que hexane, diméthylformamide, diméthylsulfoxyde, sulfolane, acétonitrile, pyridine et similaires peuvent être utilisés.

La réaction est avantageusement conduite en présence d'un agent basique, tel qu'un carbonate alcalin ou la triéthylamine, surtout dans le cas où L est un atome d'halogène.

La température de réaction peut varier entre la température ambiante (environ 20°C) et celle de reflux et les temps de réaction varient en conséquence. En général, après 6 à 12 heures de chauffage au reflux, la réaction est terminée et le produit final ainsi obtenu peut être isolé selon les techniques conventionnelles sous forme de base libre ou d'un de ses sels et la base libre est éventuellement transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Alternativement les composés de formule (I) où Y est CH, leurs sels ou solvates et leurs sels d'ammonium quaternaire, peuvent être préparés par un procédé qui prévoit

(a) de faire réagir le composé de formule (IV)

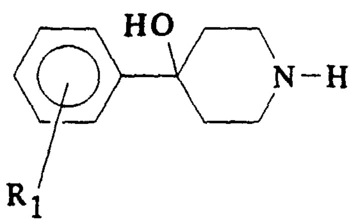

(IV)

dans laquelle $R_1$ est défini comme précédemment, avec un dérivé fonctionnel de l'acide de formule (V)

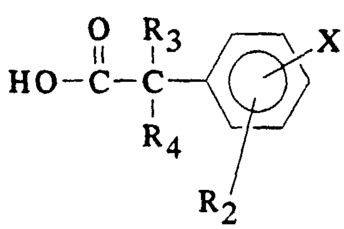

(V)

dans laquelle $R_2$ , $R_3$ , $R_4$ et X sont tels que définis ci-dessus,

(b) de réduire le groupe carbonyle du composé de formule (VI)

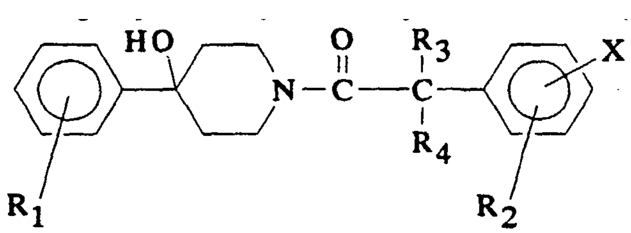

(VI)

(c) de déshydrater le pipéridinol intermédiaire de formule (VII)

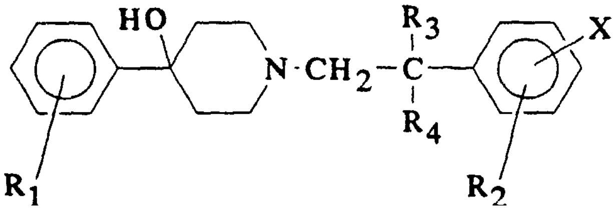
(VII)

(d) d'isoler le composé de formule (I) ainsi obtenu et, éventuellement le transformer en l'un de ses sels ou solvates ou l'un de ses sels d'ammonium quaternaire.

La réaction de l'étape (a) peut être convenablement conduite dans un solvant organique à une température comprise entre -10°C et la température de reflux du mélange réactionnel.

Comme dérivé fonctionnel approprié de l'acide de formule (V), on peut utiliser l'acide libre, éventuellement activé (par exemple avec le BOP), l'anhydride, un anhydride mixte, un ester actif ou un halogénure d'acide, le bromure de préférence. Parmi les esters actifs, l'ester de p-nitrophényle est particulièrement préféré, mais les esters de méthoxyphényle, de trityle, de benzhydryle et similaires sont également convenables.

La température de réaction peut varier entre -10°C et la température de reflux, mais en général on opère à la température ambiante ou à 30-50°C. Il peut être préférable de réaliser la réaction à froid lorsqu'elle est exothermique, comme dans le cas où on utilise le chlorure en tant que dérivé fonctionnel de l'acide de formule (V).

Comme solvant de réaction, on utilise de préférence un solvant halogéné tel que le chlorure de méthylène, le dichloroéthane, le 1,1,1-trichloroéthane, le chloroforme et similaires ou un alcool, tel que le méthanol ou l'éthanol, mais aussi d'autres solvants organiques compatibles avec les réactifs employés, par exemple le dioxane, le tétrahydrofuranne ou un hydrocarbure tel que l'hexane, peuvent être également employés.

La réaction peut être convenablement conduite en présence d'un accepteur de protons, par exemple d'un carbonate alcalin ou d'une amine tertiaire.

La réduction de l'étape (b) peut être convenablement réalisée par des agents de réduction appropriés tels que les complexes du borane, par exemple le boranediméthylsulfure, les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofuranne, le dioxane ou le 1,2-diméthoxyéthane.

Selon un mode opérationnel préférentiel, on opère avec le borane-diméthylsulfure utilisé en excès par rapport au composé (VI) de départ, à la température de reflux éventuellement sous atmosphère inerte. La réduction est normalement terminée après quelques heures.

La déshydratation de l'étape (c) est aisément conduite par exemple en utilisant un mélange acide acétique/acide sulfurique, à une température comprise entre la température ambiante et la température de reflux du solvant utilisé.

Selon une méthode préférée la réaction de l'étape (c) est conduite dans un mélange acide acétique/acide sulfurique dans un rapport de 1/3 en volume, en chauffant à la température d'environ 110°C pendant 1-3 heures.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Le composé de formule (I) obtenu est isolé selon les techniques usuelles et éventuellement transformé en un de ses sels d'addition d'acides ou, lorsqu'un groupe acide est présent, le caractère amphotère du composé permet la séparation des sels soit avec des acides soit avec des bases.

Les composés de formule (VI) et (VII), qui peuvent être regroupés dans la formule (i)

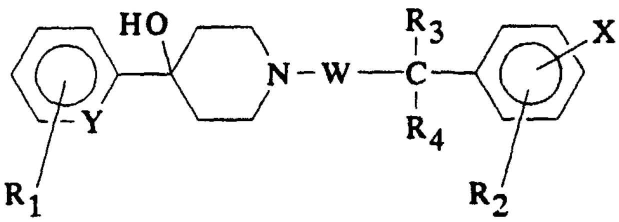
(i)

dans laquelle $R_1$, $R_2$, $R_3$, $R_4$, X et Y sont tels que définis précédemment et W représente un groupe méthylène ou un groupe carbonyle, intermédiaires clés dans la synthèse des composés de formule (I), sont des composés nouveaux et constituent un objet ultérieur de la présente invention.

Lorsque les sels du composé de formule (I) sont préparés pour être administrés en tant que médicaments, il faut que les acides ou les bases employés soient pharmaceutiquement acceptables; si l'on prépare des sels du composé de formule (I) dans un autre but, par exemple pour mieux purifier le produit ou pour mieux effectuer des essais analytiques, on peut alors utiliser n'importe quel acide ou base.

Les sels avec des bases pharmaceutiquement acceptables sont par exemple ceux avec les métaux alcalins ou alcalino-terreux, tels que le sodium, le potassium, le calcium, le magnésium et ceux avec les bases organiques, telles que les amines, les acides aminés basiques (la lysine, l'arginine, l'histidine), le trométamol, la N-méthylglutamine, etc.

Les sels avec des acides pharmaceutiquement acceptables sont par exemple, ceux avec les acides minéraux, tels que le chlorhydrate, le bromhydrate, le borate, le phosphate, le sulfate, l'hydrogénosulfate, l'hydrogénophosphate et ceux avec les acides organiques, tels que le citrate, le benzoate, l'ascorbate, le méthylsulfate, le naphtalene-2-sulfonate, le picrate, le fumarate, le maléate, le malonate, l'oxalate, le succinate, l'acétate, le tartrate, le mésylate, le tosylate, l'iséthionate, l'$\alpha$-cétoglutarate, l'$\alpha$-glycérophosphate, le glucose-1-phosphate, etc.

Les amines de départ de formule (II) où Y est CH sont des composés connus ou bien elles peuvent être préparées selon des procédés analogues à ceux utilisés pour préparer les composés connus.

Les amines de départ de formule (II) où Y est N peuvent être préparées par réaction de la 2-halogénopyridine appropriée de formule (p)

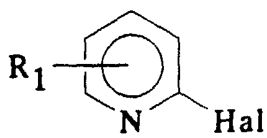

(p)

avec une 1,2,3,6-tétrahydropyridine de formule (q)

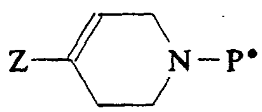

(q)

dans laquelle P• représente un groupe protecteur tel que par exemple le groupe benzyle et Z représente un substituant qui permet la substitution nucléophile de l'halogène de la pyridine. De tels substituants sont par exemple les trialkylstannanes, comme le tributylstannane ou les composés de Grignard.

On déprotège ensuite la 1,2,3,6-tétrahydropyridine par clivage du groupe protecteur dans des conditions convenables.

Les amines de formule (II') ci-dessous,

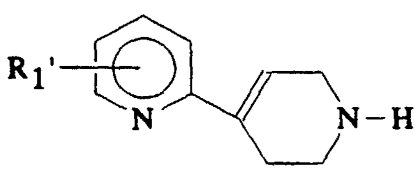

(II')

où $R_1$' représente un halogène, $CF_3$, $(C_3$-$C_4)$alkyle ou $(C_1$-$C_4)$alcoxyle, et leurs sels, sont des composés nouveaux et constituent un objet ultérieur de la présente invention.

Les composés de formule (III) peuvent être préparés

- soit par réduction des acides de formule (V) en alcool et par transformation de la fonction hydroxyle en groupe partant;
- soit, pour préparer un composé de formule (III) dans laquelle $R_3$ = $R_4$ = H, par réaction du benzène approprié de formule (r)

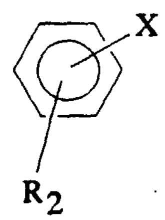

(r)

dans laquelle $R_2$ et X sont tels que définis précédemment, avec un halogénure d'acyle de formule L-$CH_2$-CO-Hal en présence d'un acide de Lewis selon la réaction bien connue de Friedel-Crafts, et par réduction de la cétone de formule (s) ainsi obtenue

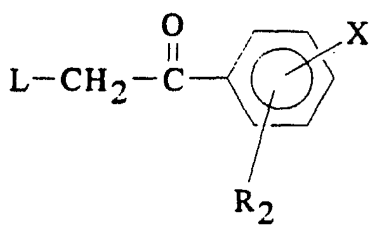

(s)

selon les méthodes opératoires largement décrites en littérature;

Les acides de formule (V) sont en général des composés décrits en littérature. Un grand nombre de ces composés sont en général décrits en tant qu'agents antiinflammatoires, comme par exemple l'hexaprofène, le tétriprofène, l'alclofénac, le butiprofène, le mexoprofène, l'ibufénac, l'ibuprofène, le flurbiprofène, le phénoprofène, le fenclofénac, etc.

Les produits de départ (III) et (V) où X est un groupe phényle, éventuellement substitué, peuvent également être préparés par un procédé original, en effectuant la réaction de Sukuzi en opérant en milieu aqueux selon un procédé nouveau qui constitue un objet ultérieur de la présente invention.

Ainsi, selon un autre de ses aspects, la présente invention concerne un procédé pour la préparation de dérivés biphénylyl par condensation entre des derivés phényl substitués par un groupe partant et des acides benzèneboroniques, en présence d'un catalyseur, d'une base forte et d'un agent de transfert de phase.

La présente invention a donc également pour objet un procédé de préparation des composés de formule (t) ci-dessous,

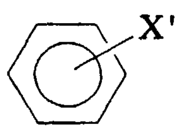

(t)

où le benzène peut être éventuellement substitué et X' est un phényle, éventuellement mono- ou polysubstitué par un halogène, $CF_3$, ($C_1$-$C_4$)alkyle, ($C_1$-$C_4$)alcoxy, cyano, amino, mono- ou di-($C_1$-$C_4$)alkylamino, ($C_1$-$C_4$)acylamino, carboxy, ($C_1$-$C_4$)alcoxycarbonyle, aminocarbonyle, mono- ou di-($C_1$-$C_4$)alkylaminocarbonyle, amino($C_1$-$C_4$)alkyle, hydroxy($C_1$-$C_4$)alkyle ou halogéno($C_1$-$C_4$)alkyle, ledit procédé consistant à faire réagir un composé de formule (w)

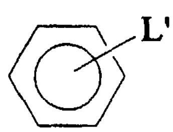

(w)

où le benzène peut être éventuellement substitué et L' représente un groupe partant tel que défini précédemment pour L, avec un acide benzèneboronique de formule X'-$B(OH)_2$ où X' est tel que défini ci-dessus, en présence d'un sel de palladium, d'une base forte et d'un agent de transfert de phase, en milieu aqueux.

Plus particulièrement et selon un aspect avantageux, la présente invention concerne un procédé de préparation des composés de formule (t')

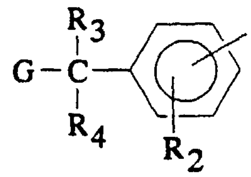

(t')

où $R_2$, $R_3$, $R_4$ et X' sont tels que définis précédemment et G représente un groupe carboxy ou L-$CH_2$- où L est un groupe partant tel que défini précédemment, qui consiste à faire réagir un composé de formule (w')

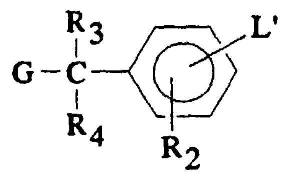

(w')

où $R_2$, $R_3$, $R_4$, G et L' sont tels que définis précédemment, avec un acide benzèneboronique de formule X'-$B(OH)_2$ où X' est tel que défini ci-dessus, en présence d'un sel de palladium, d'une base forte et d'un agent de transfert de phase, en milieu aqueux.

Des groupes partant L' préférés sont le brome et le groupe trifluorométhylsulfonyloxy.

L'acétate de palladium est un sel de palladium préféré.

Comme base forte on peut utiliser par exemple des hydroxydes ou des carbonates de métaux alcalins, tels que l'hydroxyde ou le carbonate de sodium ou l'hydroxyde ou le carbonate de potassium.

Comme agent de transfert de phase on peut utiliser les halogénures de tétraalkylammonium, le bromure de tétrabutylammonium étant particulièrement avantageux.

La réaction est avantageusement conduite en chauffant le mélange entre 30°C et le reflux, notamment entre 50°C et 80°C, de préférence à environ 70°C.

La réaction est terminée rapidement, normalement en quelques heures, selon la température à laquelle on opère.

Les produits de formule (t') et les acides de formule X'-$B(OH)_2$ sont des produits connus en littérature ou ils peuvent être préparés selon des méthodes analogues à celles utilisées pour les composés connus. Des exemples de synthèse sont quand-même consignés dans la partie expérimentale de la présente description.

L'activité des composés de formule (I) sur le système nerveux a été demontrée dans des études *in vitro* et *in vivo* selon les méthodes décrites dans EP-0 458 696 et, pour l'évaluation de la survie neuronale, à l'aide d'un test de survie *in vitro* conduit en utilisant des neurones isolés à partir de dissections de la région septale d'embryons de rats.

Plus particulièrement on a prélevé la région septale d'embryons de rats agés de 17-18 jours sous microscope à dissection dans des conditions stériles, puis on l'a dissociée dans un milieu trypsine-EDTA. La suspension de cellules a été placée dans un flacon de culture dans un milieu DME/Ham's F12 (v:v) (Dulbecco Modified Eagle Medium/ Nutrient Mixture Ham's F12 - R.G. Ham, Proc. Nat. Sci, 1965, 53:288) contenant du sérum de veau à 5% et du sérum de cheval à 5% et maintenue à 37°C pendant 90 minutes. Ce traitement permet l'élimination des cellules non-neuronales.

Les neuroblastes sont ensuite ensemencés dans les puits d'une plaque de titration à raison de $17x10^4$ cellules/$cm^2$, dans un milieu de culture non sérique constitué par du DME/Ham's F12 contenant du sélénium (30 nM) et de la transferrine (1,25 µM). Chaque puits a été préalablement traité à la poly-L-lysine. Les plaques ensemencées sont placées dans un incubateur à l'étuve (37°C; 5% $CO_2$).

Les composés à tester sont dissouts dans du DMSO et dilués comme requis par le milieu de culture.

Les neuroblastes sont maintenus dans des plaques contenant le composé à tester ou le solvant correspondant pendant 4 jours sans changer le milieu.

Après 4 jours le milieu est remplacé par un sel de tétrazolium dissout dans le milieu de culture (0,15 mg/ml). Les cellules sont ensuite placées à l'étuve à 37°C pendant 4 heures. Les succinodéshydrogénases mitochondriales des cellules vivantes réduisent le sel de tétrazolium en bleu formazan dont, après dissolution dans le DMSO, on mesure la densité optique à 540 nm, densité qui est linéairement corrélée au nombre de cellules vivantes (Manthorpe et al., Dev. Brain Res., 1988, 25:191-198).

La différence entre les groupes contenant les composés à tester et les témoins a été évaluée par analyse statistique en utilisant le test t bilatéral de Dunnett ("two-tailed Dunnett t-test").

Dans ce dernier test les composés de formule (I) se sont montrés aussi actifs ou plus actifs que les composés

décrits dans EP-0 458 696, l'efficacité de certains composés de formule (I) vis-à-vis de la survie neuronale étant double par rapport au composé A décrit dans EP-0 458 696.

Grâce à cette puissante activité neuroprotectrice, et à leur faible toxicité compatible pour une utilisation en tant que médicaments, les composés de formule (I) ainsi que leurs sels d'addition pharmaceutiquement acceptables, leurs solvates et leurs sels d'ammonium quaternaire sont utilisables pour la préparation de compositions pharmaceutiques indiquées dans le traitement et/ou la prophylaxie de toutes les maladies qui impliquent une dégénérescence neuronale. Plus particulièrement, les composés de l'invention sont utilisables, seuls ou en co-administration ou association avec d'autres principes actifs agissant sur le SNC, par exemple les cholinomimétiques M1 sélectifs, les antagonistes NMDA, les nootropiques tels que le piracétam, notamment dans les indications suivantes: troubles de la mémoire, démence vasculaire, troubles post-encéphalitiques, troubles post-apoplectiques, syndromes post-traumatiques dûs à un traumatisme crânien, troubles dérivant d'anoxies cérébrales, maladie d'Alzheimer, démence sénile, démence subcorticale, telle que la chorée de Huntington et la maladie de Parkinson, démence provoquée par le SIDA, neuropathies dérivées de morbidité ou dommage des nerfs sympathiques ou sensoriels, maladies cérébrales, telles que l'oedème cérébral, les dégénérescences spinocérébelleuses, et les dégénérescences des motoneurones, comme par exemple la sclérose latérale amyotrophique.

Selon un autre aspect, l'invention concerne une méthode de traitement des affections sus-mentionnées, qui consiste à administrer à un patient en ayant besoin une quantité thérapeutiquement efficace d'un composé selon l'invention, tel quel ou en mélange avec des supports pharmaceutiques classiques.

L'administration des composés de l'invention peut être convenablement effectuée par voie orale, parentérale, sublinguale ou transdermique. La quantité de principe actif à administrer dans le traitement des troubles cérébraux et neuronaux selon la méthode de la présente invention dépend de la nature et de la gravité des affections à traiter ainsi que du poids des malades. Néanmoins, les doses unitaires préférées comprendront généralement de 0,5 à 700 mg, avantageusement de 2 à 300 mg, de préférence de 5 à 150 mg, par exemple entre 5 et 50 mg, à savoir 1, 2, 5, 10, 15, 20, 25, 30, 40 ou 50 mg, de produit. Ces doses unitaires seront administrées normalement une ou plusieurs fois par jour, par exemple 2, 3, 4, ou 5 fois par jour, de préférence une à trois fois par jour, la dose globale chez l'homme étant variable entre 1 et 1400 mg par jour, avantageusement entre 2 et 900 mg par jour, par exemple de 3 à 500 mg, plus convenablement de 10 à 300 mg par jour.

Dans les compositions pharmaceutiques de la présente invention pour l'administration orale, sublinguale, sous-cutanée, intramusculaire, intraveineuse, transdermique ou rectale, le principe actif peut être administré sous formes unitaires d'administration, soit tel quel par exemple sous forme lyophilisée, soit en mélange avec des supports pharmaceutiques classiques, aux animaux et aux êtres humains pour le traitement des affections susdites. Les formes unitaires d'administration appropriées comprennent les formes par voie orale telles que les comprimés éventuellement sécables, les gélules, les poudres, les granules et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Les exemples qui suivent illustrent mieux l'invention sans toutefois la limiter.

**PREPARATION 1**

**Acide 2-trifluorométhylbenzèneboronique.**

On mélange, sous atmosphère d'argon, 1,36 ml (0,01 mmole) de 2-bromotrifluorométhylbenzène et 10 ml d'éther éthylique anhydre. On refroidit à 0°C et on y ajoute 7 ml de BuLi (solution 1,6 M dans hexane) et on laisse à la température de 0°C pendant 2 heures. On transfère ensuite le mélange dans une solution à - 78°C, sous atmosphère d'argon, de 3 ml de triisopropylborate (0,0125 mole) et 15 ml de THF anhydre.
On laisse à - 78°C pendant 3 heures, puis une nuit à la température ambiante. On verse dans une solution aqueuse 1N d'acide chlorhydrique, on extrait à l'éther éthylique, on sèche la phase organique sur du sulfate de sodium et on évapore le solvant sous pression réduite.
On traite le résidu avec de l'hexane et on filtre le précipité formé. On obtient 0,7 g du composé du titre. P.f. 140-143°C.

**PREPARATION 2**

**Acide 3-chlorobenzèneboronique.**

En opérant comme décrit dans la préparation 1, mais en utilisant le 3-bromochlorobenzène au lieu du 2-bromotrifluorométhylbenzène, on obtient le composé du titre. P.f. 176-178°C.

**PREPARATION 3**

***Acide (2-chloro-4-méthoxyphényl)acétique.***

***3i/ 2-chloro-4-méthoxy-acétophénone.***

A un mélange de 9,3 ml (0,13 mole) de chlorure d'acétyle dans 350 ml de chlorure de méthylène, à 0°C, on ajoute 14,3 g (0,11 mole) de 3-chloroanisole. On agite le mélange pendant 1 heure puis, tout en maintenant la température à 0°C, on ajoute 24 g (0,18 mole) de chlorure d'aluminium et on laisse réagir à cette température pendant 2 heures. On ajoute ensuite 20 ml d'une solution 1N d'acide chlorhydrique et 20 ml d'eau. On sépare les deux phases, on lave la phase organique à l'eau, on la sèche sur du sulfate de sodium, on filtre et on évapore le solvant sous pression réduite. On purifie le brut par chromatographie sur colonne de gel de silice en éluant avec un mélange cyclohexane/acétate d'éthyle = 8/2. On obtient le composé du titre.

***3ii/ 4-(2-chloro-4-méthoxy-benzylthiocarbonyl)morpholine.***

On chauffe à 130°C pendant 4 heures, un mélange de 5,52 g (0,03 mole) du produit de l'étape précédente, 7,1 ml de morpholine et 1,15 g de soufre. On ajoute ensuite une solution 1N d'acide chlorhydrique et on extrait à l'acétate d'éthyle. On sépare les deux phases, on sèche la phase organique sur du sulfate de sodium et on évapore le solvant sous pression réduite. On traite le brut avec 2,5 ml d'acétate d'éthyle et on filtre le précipité formé. On obtient le composé du titre. P.f. 100-102°C.

***3iii/ Acide (2-chloro-4-méthoxyphényl)acétique.***

On chauffe au reflux pendant 8 heures un mélange de 6,6 g du produit de l'étape précédente, 35 ml d'éthanol et une solution de 3 g d'hydroxyde de sodium dans 55 ml d'eau. On lave à l'éther éthylique, on acidifie la solution aqueuse avec de l'acide chlorhydrique 1N et on extrait à l'éther éthylique. On sèche la phase organique sur du sulfate de sodium, on filtre et on évapore le solvant sous pression réduite. On obtient le composé du titre. P.f. 115-117°C.

**PREPARATION 4**

***Acide (2-chloro-4-hydroxyphényl)acétique.***

On chauffe au reflux pendant deux heures une solution de 1,6 g (8 mmole) de l'acide (2-chloro-4-méthoxyphényl)acétique préparé selon la préparation 3, dans 13 ml d'acide bromhydrique (en solution aqueuse à 48%). Après refroidissement, on ajoute de l'hydroxyde d'ammonium jusqu'à pH basique et on extrait au chlorure de méthylène. On évapore l'eau de la phase aqueuse et on lave le résidu avec de l'éthanol plusieurs fois. On obtient le composé du titre.

EXEMPLE 1

**Chlorhydrate de 1-[2-(4-isobutylphényl)propyl]-4-(3-trifluorométhylphényl)-1,2,3,6-tétrahydropyridine.**

**1a/ *Ester éthylique de l'acide 2-(4-isobutylphényl)propionique.***

Dans une solution de 50 g (0,242 mole) d'acide 2-(4-isobutylphényl)propionique dans 700 ml d'éthanol absolu, on fait barboter de l'acide chlorhydrique gazeux pendant une heure puis on chauffe au reflux pendant deux heures. On évapore le solvant et on reprend le résidu dans de l'acétate d'éthyle. On lave avec une solution aqueuse de bicarbonate de sodium, on sèche sur du sulfate de sodium et on évapore le solvant sous pression réduite. On obtient le composé du titre.

**1b/ *Alcool 2-(4-isobutylphényl)propylique.***

A une suspension de 9,55 g d'hydrure de lithium-aluminium dans 50 ml d'éther éthylique, sous azote, on ajoute une solution de 59 g (0,25 mole) du composé de l'étape précédente dans 450 ml d'éther éthylique, en maintenant la température à 20°C. On agite à la température ambiante pendant 2 heures puis on ajoute au mélange réactionnel une solution de 60 ml d'éthanol 95% et 60 ml d'eau pour détruire, avec extrème précaution, l'hydrure n'ayant pas réagi. On filtre les sels formés et on évapore le filtrat sous pression réduite. On obtient le composé du titre.

**1c/ *2-(4-isobutylphényl)propyl méthanesulfonate.***

On refroidit un mélange de 15,5 g (0,08 mole) du composé de l'étape précédente, 100 ml de chlorure de méthylène et 24,2 g (0,1774 mole) de triéthylamine à 0-5°C et on ajoute une solution de 12,9 g (0,1774 mole) de chlorure de mésyle dans 10 ml de chlorure de méthylène. On agite à la température ambiante pendant 5 heures. On lave ensuite la solution avec une solution d'acide chlorhydrique 1N, à l'eau, avec une solution aqueuse de bicarbonate de sodium et encore à l'eau. On sèche la phase organique sur du sulfate de sodium et on évapore le solvant sous pression réduite. On obtient le composé du titre.

**1d/ *Chlorhydrate de 1-[2-(4-isobutylphényl)propyl]-4-(3-trifluorométhylphényl)-1,2,3,6-tétrahydropyridine.***

On chauffe au reflux pendant une nuit un mélange de 2,63 g (0,01 mole) de 4-(3-trifluorométhylphényl)-1,2,3,6-tétrahydropyridine, 4,2 ml (0,03 mole) de triéthylamine, 4 g (0,01 mole) de 2-(4-isobutylphényl)-1-(méthylsulfonyloxy)propane dans 40 ml d'isopropanol. On évapore le solvant sous pression réduite et on purifie le résidu par chromatographie sur colonne de gel de silice en éluant avec un mélange cyclohexane/acétate d'éthyle = 8/2. On prépare le chlorhydrate de l'huile ainsi obtenue à l'aide d'une solution d'acide chlorhydrique dans l'éther éthylique. On obtient un précipité que l'on filtre puis cristallise dans de l'acétone. P.f. 190-192°C.

EXEMPLE 2

**Chlorhydrate de 1-[(2S)-2-(4-isobutylphényl)propyl]-4-(3-trifluorométhylphényl)-1,2,3,6-tétrahydropyridine.**

**2a/ *1-[(2S)-2-(4-isobutylphényl)propionyl]-4-hydroxy-4-(3-trifluorométhylphényl)pipéridine.***

On mélange 2,06 g (0,01 mole) d'acide (2S)-2-(4-isobutylphényl)propionique, 35 ml de chlorure de méthylène, 2,09 g (0,015 mole) de triéthylamine, 2,45 g (0,01 mole) de 4-(3-trifluorométhylphényl)-4-pipéridinol, 4,42 g (0,01 mole) de BOP et on agite la solution à la température ambiante pendant 1,5 heures. On ajoute ensuite de l'acétate d'éthyle et on lave à l'eau, à l'acide chlorhydrique 1N, à l'eau, à l'hydroxyde de sodium 1N, à l'eau. On sèche la phase organique sur du sulfate de sodium et on évapore le solvant sous pression réduite. On purifie l'huile brute ainsi obtenue par chromatographie sur colonne de gel de silice en éluant avec un mélange acétate d'éthyle/cyclohexane = 9/1. On isole 3,3 g du produit du titre. P.f. 123-125°C. $[\alpha]_D^{20}$ = -66,7° (c = 1%, MeOH).

**2b/ *Chlorhydrate de 1-[(2S)-2-(4-isobutylphényl)propyl]-4-hydroxy-4-(3-trifluorométhylphényl)pipéridine.***

On chauffe au reflux 3,0 g (0,0069 mole) du produit de l'étape précédente dans 30 ml de tétrahydrofuranne et on y ajoute 2,09 ml (0,02 mole) de boranediméthylsulfure dans 20 ml de tétrahydrofuranne. On chauffe au reflux pendant 4 heures, on refroidit la solution à 10-15°C et on y ajoute goutte à goutte, avec précaution, 15 ml de méthanol. On agite 15 minutes à la température ambiante et ensuite 30 minutes au reflux. On évapore le solvant sous pression

réduite, on reprend avec une solution d'ammoniaque diluée. On extrait à l'acétate d'éthyle, on lave la phase organique à l'eau, on sèche sur du sulfate de sodium et on évapore le solvant sous pression réduite. On obtient 3,0 g d'huile brute. On prépare le chlorhydrate par traitement avec une solution d'isopropanol saturée en acide chlorhydrique. On obtient le composé du titre. P.f. 250-252°C. $[\alpha]_D^{20}$ = +35,01° (c = 1%, MeOH).

**2c/ *Chlorhydrate de 1-[(2S)-2-(4-isobutylphényl)propyl]-4-(3-trifluorométhylphényl)-1,2,3,6-tétrahydropyridine.***

On dissout 1,3 g (0,0023 mole) du produit de l'étape précédente dans 10 ml d'acide acétique, on y ajoute 3 ml d'acide sulfurique à 96% et on chauffe à 110°C pendant 2 heures. On verse dans un mélange eau/glace, on ajoute une solution d'hydroxyde de sodium concentrée et on extrait à l'éther éthylique. On lave la phase organique à l'eau, on sèche sur du sulfate de sodium et on évapore le solvant sous pression réduite. On obtient 1,25 g d'huile brute. On prépare le chlorhydrate par traitement avec une solution d'isopropanol saturée en acide chlorhydrique. On obtient le composé du titre qu'on cristallise dans de l'acétone. P.f. 223-225°C. $[\alpha]_D^{20}$ = +46,8° (c = 1%, MeOH).

EXEMPLE 3

**Chlorhydrate de 1-[(2R)-2-(4-isobutylphényl)propyl]-4-(3-trifluorométhylphényl)-1,2,3,6-tétrahydropyridine.**

**3a/ *1-[(2R)-2-(4-isobutylphényl)propionyl]-4-hydroxy-4-(3-trifluorométhylphényl)pipéridine.***

En opérant comme décrit dans l'exemple 2a, mais en utilisant l'acide (2R)-2-(4-isobutylphényl)propionique, on obtient le produit du titre. P.f. 122-124°C. $[\alpha]_D^{20}$ = +68,7° (c = 1%, MeOH).

**3b/ *Chlorhydrate de 1-[(2R)-2-(4-isobutylphényl)propyl]-4-hydroxy-4-(3-trifluorométhylphényl)pipéridine.***

En opérant comme décrit dans l'exemple 2b, en utilisant le composé de l'étape 3a, on obtient le produit du titre. P.f. 257-260°C. $[\alpha]_D^{20}$ = -37,2° (c = 1%, MeOH).

**3c/ *Chlorhydrate de 1-[(2R)-2-(4-isobutylphényl)propyl]-4-(3-trifluorométhylphényl)-1,2,3,6-tétrahydropyridine.***

En opérant comme décrit dans l'exemple 2c, en utilisant le composé de l'étape 3b, on obtient le produit du titre. P.f. 224-226°C. $[\alpha]_D^{20}$ = -45,6° (c = 1%, MeOH).

EXEMPLE 4

**Chlorhydrate de 1-[2-(4-isobutylphényl)éthyl]-4-(3-trifluorométhylphényl)-1,2,3,6-tétrahydropyridine.**

**4a/ *1-bromo-2-(4-isobutylphényl)éthane.***

On refroidit à 0-5°C un mélange de 5 g (0,0373 mole) d'isobutylbenzène, 67 ml de chlorure de méthylène, 9,86 g (0,0489 mole) de bromoacétylbromure et on y ajoute 5,75 g (0,0431 mole) de trichlorure d'aluminium. On agite à 0-5°C pendant une heure puis on laisse une nuit à la température ambiante. On verse dans un mélange eau/glace, on extrait au chlorure de méthylène, on sèche la phase organique sur du sulfate de sodium et on évapore le solvant sous pression réduite. On mélange 4,6 g (0,018 mole) de l'huile ainsi obtenue avec 9,7 ml (0,123 mole) d'acide trifluoroacétique et 12,7 ml (0,0792 mole) de triéthylsilane et on chauffe à 80°C pendant 4 heures. On ajoute ensuite une solution aqueuse saturée de bicarbonate de sodium jusqu'à pH basique, on extrait à l'éther éthylique, on sèche la phase organique sur du sulfate de sodium et on évapore le solvant sous pression réduite. On purifie l'huile brute ainsi obtenue par chromatographie sur colonne de gel de silice en éluant avec du cyclohexane. On obtient le composé du titre. Chromatographie sur couche mince (éluant cyclohexane) : R.f. 0,5.

**4b/ *Chlorhydrate de 1-[2-(4-isobutylphényl)éthyl]-4-(3-trifluorométhylphényl)-1,2,3,6-tétrahydropyridine.***

On chauffe au reflux pendant 8 heures un mélange de 2,5 g (0,0095 mole) de 4-(3-trifluorométhylphényl)-1,2,3,6-tétrahydropyridine, 50 ml de butanol, 3,94 g (0,0285 mole) de carbonate de potassium anhydre râpé et 2,3 g (0,0095 mole) du produit de l'étape précédente. On évapore le solvant sous pression réduite, on reprend à l'acétate d'éthyle, on lave à l'eau, on sèche sur du sulfate de sodium et on évapore le solvant sous pression réduite. On prépare le chlorhydrate de l'huile ainsi obtenue par traitement avec une solution d'isopropanol saturée en acide chlorhydrique. On obtient 2,4 g du composé du titre qu'on cristallise dans de l'isopropanol. P.f. 242-246°C. Chromatographie sur

couche mince (éluant cyclohexane/acétate d'éthyle = 1/1) : R.f. 0,6.

EXEMPLE 5

**Chlorhydrate de 1-[2-(4-tert-butylphényl)éthyl]-4-(3-trifluorométhylphényl)-1,2,3,6-tétrahydropyridine.**

**5a/** ***1-bromo-2-(4-tert-butylphényl)éthane.***

En opérant comme décrit dans l'exemple 4a, mais en utilisant le tert-butylbenzène au lieu de l'isobutylbenzène, on obtient le produit du titre. Chromatographie sur couche mince (éluant cyclohexane) : R.f. 0,6.

**5b/** ***Chlorhydrate de 1-[2-(4-tert-butylphényl)éthyl]-4-(3-trifluorométhylphényl)-1,2,3,6-tétrahydropyridine.***

En opérant comme décrit dans l'exemple 4b, en utilisant le composé de l'étape Sa, on obtient le composé du titre. P.f. 261-265°C. Chromatographie sur couche mince (éluant cyclohexane/acétate d'éthyle = 1/1): R.f. 0,7.

EXEMPLE 6

**Oxalate de 1-[2-(4-isobutylphényl)-2-méthylpropyl]-4-(3-trifluorométhylphényl)-1,2,3,6-tétrahydropyridine.**

**6a/** ***Acide 2-(4-isobutylphényl)-2-méthylpropionique.***

On dissout 5 g (0,0227 mole) d'ester éthylique de l'acide 2-(4-isobutylphényl)-propionique (Exemple 1a) dans 50 ml de DMF et on y ajoute par portions 1,6 g (0,025 mole) d'hydrure de sodium. Après 30 minutes on y ajoute 3,2 ml (0,0375 mole) d'iodure de méthyle. On agite à la température ambiante pendant 2 heures, on verse dans un mélange eau/glace, on extrait à l'acétate d'éthyle, on lave à l'eau, on sèche sur du sulfate de sodium et on évapore le solvant sous pression réduite. On mélange 5,7 g de l'huile ainsi obtenue avec 5,7 g d'hydroxyde de potassium, 35 ml d'eau et 35 ml d'éthanol absolu et on chauffe au reflux pendant 2 heures. On évapore l'éthanol sous pression réduite, on lave à l'acétate d'éthyle, on acidifie la phase aqueuse avec de l'acide chlorhydrique, on extrait à l'acétate d'éthyle, on sèche la phase organique sur du sulfate de sodium et on évapore le solvant. On obtient le composé du titre sous forme d'huile semi-solide.

**6b/** ***1-[2-(4-isobutylphényl)-2-méthylpropionyl]-4-hydroxy-4-(3-trifluorométhylphényl)pipéridine.***

On mélange 2,86 g (0,013 mole) de l'acide de l'étape précédente, 45 ml de chlorure de méthylène, 2,7 ml (0,0195 mole) de triéthylamine, 3,18 g (0,013 mole) de 4-hydroxy-4-(3-trifluorométhylphényl)pipéridine et 5,74 g (0,013 mole) de BOP et on agite à la température ambiante pendant 1,5 heures. On évapore le solvant sous pression réduite, on reprend à l'acétate d'éthyle, on lave à l'eau, à l'acide chlorhydrique 1N, à l'eau, à l'hydroxyde de sodium 1N, à l'eau, on sèche la phase organique sur du sulfate de sodium, on filtre et on évapore le solvant. On obtient une huile qu'on cristallise dans de l'éther isopropylique. On obtient 1,5 g du composé du titre. P.f. 158-160°C. Chromatographie sur couche mince (éluant cyclohexane/acétate d'éthyle = 7/3) : R.f. 0,4.

**6c/** ***Chlorhydrate de 1-[2-(4-isobutylphényl)-2-méthylpropyl]-4-hydroxy-4-(3-trifluorométhylphényl)pipéridine.***

On chauffe au reflux 2,78 g (0,00622 mole) du composé de l'étape précédente dans 30 ml de tétrahydrofuranne et on y ajoute 1,9 ml (0,0186 mole) de boranediméthylsulfure dans 20 ml de tétrahydrofuranne, en maintenant le reflux pendant 4 heures. On refroidit la solution à 10-15°C, on y ajoute 15 ml de méthanol, on agite 15 minutes à la température ambiante et 30 minutes au reflux. On évapore le solvant sous pression réduite, on reprend le résidu à l'eau (15 ml), on y ajoute de l'hydroxyde d'ammonium concentré jusqu'à pH basique, on extrait à l'acétate d'éthyle, on lave à l'eau, on sèche la phase organique sur du sulfate de sodium et on évapore le solvant sous pression réduite. On obtient 3 g d'huile qu'on salifie par traitement avec de l'isopropanol saturé en acide chlorhydrique. On obtient 1,4 g du composé du titre. P.f. 263-265°C. Chromatographie sur couche mince (éluant cyclohexane/acétate d'éthyle = 7/3) : R. f. 0,3 (base).

**6d/** ***Oxalate de 1-[2-(4-isobutylphényl)-2-méthylpropyl]-4-(3-trifluorométhylphényl)-1,2,3,6-tétrahydropyridine.***

On chauffe au reflux pendant 2 heures 1,2 g (0,0025 mole) du composé de l'étape précédente, 10 ml d'acide acétique glacial, 3 ml d'acide sulfurique à 96%. On verse dans un mélange eau/glace, on y ajoute une solution d'hydroxyde de sodium à 20% jusqu'à pH basique, on extrait à l'acétate d'éthyle, on lave la phase organique à l'eau, on la sèche sur du sulfate de sodium et on évapore le solvant sous pression réduite. On obtient une huile qu'on traite avec de l'acide oxalique dans de l'isopropanol. On obtient ainsi le composé du titre. P.f. 164-165°C.

EXEMPLE 7

**Chlorhydrate de 1-[2-(4-isopropylphényl)éthyl]-4-(3-trifluorométhylphényl)-1,2,3,6-tétrahydropyridine.**

**7a/ *1-bromo-2-(4-isopropylphényl)éthane.***

On refroidit à 0-5°C un mélange de 4,6 g (0,030 mole) de cumène, 50 ml de chlorure de méthylène, 2,86 ml (0,033 mole) de bromoacétylbromure et on y ajoute 4 g (0,030 mole) de trichlorure d'aluminium. On agite à 0-5°C pendant une heure puis on agite pendant 4 heures à la température ambiante. On verse dans un mélange eau/glace, on extrait au chlorure de méthylène, on sèche la phase organique sur du sulfate de sodium et on évapore le solvant sous pression réduite. On mélange 8,5 g de l'huile ainsi obtenue avec 19 ml (0,246 mole) d'acide trifluoroacétique et 24,6 ml (0,154 mole) de triéthylsilane et on chauffe à 80°C pendant 4 heures. On ajoute ensuite une solution aqueuse saturée de bicarbonate de sodium jusqu'à pH basique, on extrait à l'acétate d'éthyle, on sèche la phase organique sur du sulfate de sodium et on évapore le solvant sous pression réduite. On obtient 10 g du composé du titre.

**7b/ *Chlorhydrate de 1-[2-(4-isopropylphényl)éthyl]-4-(3-trifluorométhylphényl)-1,2,3,6-tétrahydropyridine.***

On chauffe au reflux pendant 5 heures un mélange de 3,7 g (0,014 mole) de 4-(3-trifluorométhylphényl)-1,2,3,6-tétrahydropyridine, 74 ml de butanol, 4,8 g (0,035 mole) de carbonate de potassium anhydre rapé et 4 g (0,018 mole) du produit de l'étape précédente. On évapore le solvant sous pression réduite, on reprend à l'acétate d'éthyle, on lave à l'eau, on sèche sur du sulfate de sodium et on évapore le solvant sous pression réduite. On prépare le chlorhydrate de l'huile ainsi obtenue par traitement avec une solution d'isopropanol saturée en acide chlorhydrique. On obtient 2 g du composé du titre qu'on cristallise dans de l'isopropanol. P.f. 262-263°C. Chromatographie sur couche mince (éluant cyclohexane/acétate d'éthyle = 1/1) : R.f. 0,7.

EXEMPLE 8

**1-[2-(3'-chloro-4-biphénylyl)éthyl]-4-(3-trifluorométhylphényl)-1,2,3,6-tétrahydropyridine.**

**8a/ *1-bromo-2-(3'-chloro-4'-biphénylyl)éthane.***

On refroidit à 0-5°C un mélange de 5 g (0,026 mole) de 3-chloro-biphényle, 50 ml de chlorure de méthylène, 6,95 g (0,034 mole) de bromoacétylbromure et on y ajoute 4 g (0,030 mole) de trichlorure d'aluminium. On agite pendant 4 heures à la température ambiante. On verse dans un mélange eau/glace, on extrait au chlorure de méthylène, on lave la phase organique avec une solution 1N de HCl, on sèche sur du sulfate de sodium et on évapore sous pression réduite. On mélange 7 g de l'huile ainsi obtenue avec 12,1 ml (0,156 mole) d'acide trifluoroacétique et 15,8 ml (0,0986 mole) de triéthylsilane et on chauffe à 80°C pendant 4 heures. On ajoute ensuite une solution aqueuse saturée de bicarbonate de sodium jusqu'à pH basique, on extrait à l'éther éthylique, on lave la phase organique avec une solution de bicarbonate de sodium, on sèche la phase organique sur du sulfate de sodium et on évapore le solvant sous pression réduite. On obtient le composé du titre.

**8b/ *1-[2-(3'-chloro-4-biphénylyl)éthyl]-4-(3-trifluorométhylphényl)-1,2,3,6-tétrahydropyridine.***

On chauffe au reflux pendant 5 heures un mélange de 2,63 g (0,010 mole) de 4-(3-trifluorométhylphényl)-1,2,3,6-tétrahydropyridine, 80 ml de butanol, 3,5 g (0,025 mole) de carbonate de potassium anhydre râpé et 2,63 g du produit de l'étape précédente. On élimine les sels par filtration, on évapore le solvant sous pression réduite, on reprend à l'acétate d'éthyle, on lave à l'eau, on sèche sur du sulfate de sodium et on évapore le solvant sous pression réduite. On purifie le produit brut par chromatographie sur colonne de gel de silice en éluant avec un mélange acétate d'éthyle/cyclohexane = 3/7. R.f. 0,5. On obtient 2 g du composé du titre. P.f. 85-87°C.

EXEMPLE 9

**Chlorhydrate de 1-[2-(2'-chloro-4-biphénylyl)éthyl]-4-(3-trifluorométhylphényl)-1,2,3,6-tétrahydropyridine.**

**9a/ *1-bromo-2-(2'-chloro-4-biphénylyl)éthane.***

En opérant comme décrit dans l'exemple 8a, mais en utilisant 5 g de 2-chlorobiphényle au lieu du 3-chlorobiphényle, on obtient 8 g du composé du titre.

**9b/ *Chlorhydrate de 1-[2-(2'-chloro-4-biphénylyl)éthyl]-4-(3-trifluorométhylphényl)-1,2,3,6-tétrahydropyridine.***

En opérant comme décrit dans l'exemple 8b, mais en utilisant le composé de l'étape 9a, on obtient la 1-[2-(2'-chloro-4-biphénylyl)éthyl]-4-(3-trifluorométhylphényl)-1,2,3,6-tétrahydropyridine brute qu'on purifie par chromatographie sur colonne de gel de silice en éluant avec un mélange acétate d'éthyle/cyclohexane = 2/8; R.f. 0,5. On prépare le chlorhydrate à l'aide d'isopropanol saturé en acide chlorhydrique. On cristallise le produit ainsi obtenu dans de l'isopropanol. On obtient 5 g du composé du titre. P.f. 227-230°C.

EXEMPLE 10

**1-[2-(4'-chloro-4-biphénylyl)éthyl]-4-(3-trifluorométhylphényl)-1,2,3,6-tétrahydropyridine.**

**10a/ *1-bromo-2-(4'-chloro-4-biphénylyl)éthane.***

En opérant comme décrit dans l'exemple 8a, mais en utilisant 5 g de 4-chlorobiphényle au lieu du 3-chlorobiphényle, on obtient le composé du titre.

**10b/ *1-[2-(4'-chloro-4-biphénylyl)éthyl]-4-(3-trifluorométhylphényl)-1,2,3,6-tétrahydropyridine.***

En opérant comme décrit dans l'exemple 8b, mais en utilisant le composé de l'étape 10a, on obtient le composé du titre brut qu'on purifie par chromatographie sur colonne de gel de silice en éluant avec un mélange acétate d'éthyle/cyclohexane = 2/8; R.f. 0,5. On obtient 4 g du composé du titre sous forme de solide blanc qu'on cristallise dans de l'acétate d'éthyle. P.f. 146-148°C.

EXEMPLE 11

**Chlorhydrate de 1-[2-(4'-fluoro-4-biphénylyl)éthyl]-4-(3-trifluorométhylphényl)-1,2,3,6-tétrahydropyridine.**

**11a/ *1-bromo-2-(4'-fluoro-4-biphénylyl)éthane.***

En opérant comme décrit dans l'exemple 8a, mais en utilisant le 4-fluorobiphényle au lieu du 3-chlorobiphényle, on obtient le composé du titre.

**11b/ *Chlorhydrate de 1-[2-(4'-fluoro-4-biphénylyl)éthyl]-4-(3-trifluorométhylphényl)-1,2,3,6-tétrahydropyridine.***

En opérant comme décrit dans l'exemple 8b, mais en utilisant le composé de l'étape 11a, on obtient la 1-[2-(4'-fluoro-4-biphénylyl)éthyl]-4-(3-trifluorométhylphényl)-1,2,3,6-tétrahydropyridine brute. On prépare le chlorhydrate à l'aide d'isopropanol saturé en HCl. On obtient le composé du titre qu'on cristallise dans de l'isopropanol. P.f. 257-259°C. Chromatographie sur couche mince (éluant cyclohexane/acétate d'éthyle = 1/1): R.f. 0,5.

EXEMPLE 12

**Chlorhydrate de 1-[2-(3'-trifluorométhyl-4-biphénylyl)éthyl]-4-(3-trifluorométhylphényl)-1,2,3,6-tétrahydropyridine.**

En opérant comme décrit dans l'exemple 8 mais en partant du 3-trifluorométhylbiphényle au lieu du 3-chlorobiphényle, on obtient le composé du titre. P.f. 229-233 °C.

EXEMPLE 13

**Chlorhydrate de 1-[2-(4-cyclohexylphényl)éthyl]-4-(3-trifluorométhylphényl)-1,2,3,6-tétrahydropyridine.**

**13a/ *1-bromo-2-(4-cyclohexylphényl)éthane.***

En opérant comme décrit dans l'exemple 8a, mais en utilisant 10 g de cyclohexylbenzène au lieu du 3-chlorobiphényle, on obtient 5,56 g du composé du titre.

**13b/ *Chlorhydrate de 1-[2-(4-cyclohexylphényl)éthyl]-4-(3-trifluorométhylphényl)-1,2,3,6-tétrahydropyridine.***

En opérant comme décrit dans l'exemple 8b, mais en utilisant le composé de l'étape 13a, on obtient la 1-[2-(4-cyclohexylphényl)éthyl]-4-(3-trifluorométhylphényl)-1,2,3,6-tétrahydropyridine brute qu'on purifie par chromatographie sur colonne de gel de silice en éluant avec un mélange cyclohexane/acétate d'éthyle = 9/1. On prépare le chlorhydrate à l'aide d'isopropanol saturé en acide chlorhydrique. On obtient 2,55 g du composé du titre. P.f. 255-260°C. Chromatographie sur couche mince (éluant cyclohexane/acétate d'éthyle = 7/3) : R.f. 0,5.

EXEMPLE 14

**Oxalate de 1-[2-(4-biphénylyl)-2-éthyl]-4-(4-fluorophényl)-1,2,3,6-tétrahydropyridine.**

On chauffe au reflux pendant 5 heures un mélange de 1 g (0,0047 mole) de 4-(4-fluorophényl)-1,2,3,6-tétrahydropyridine, 20 ml de butanol, 1,6 g (0,012 mole) de carbonate de potassium anhydre rapé et 1,2 g de 1-bromo-2-(4-biphénylyl)éthane. On élimine les sels par filtration, on évapore le solvant sous pression réduite, on reprend à l'acétate d'éthyle, on lave à l'eau, on sèche sur du sulfate de sodium et on évapore le solvant sous pression réduite. On prépare l'oxalate de l'huile ainsi obtenue par traitement avec de l'acide oxalique dans de l'acétone. On obtient ainsi le composé du titre qu'on cristallise dans de l'éthanol. P.f. 211-215°C. Chromatographie sur couche mince (éluant: acétate d'éthyle/cyclohexane = 1/1): R.f. 0,6.

EXEMPLE 15

**Chlorhydrate de 1-[2-(4-biphénylyl)-2-méthylpropyl]-4-(3-trifluorométhylphényl)-1,2,3,6-tétrahydropyridine.**

**15a/ *Ester éthylique de l'acide 4-biphénylylacétique.***

On dissout 45 g d'acide 4-biphénylylacétique (0,212 mole) dans 650 ml d'éthanol absolu. On fait barboter dans la solution de l'acide chlorhydrique gazeux pendant 1 heure puis on chauffe la solution au reflux pendant 2 heures. On évapore l'éthanol et on reprend le résidu à l'acétate d'éthyle, on lave avec une solution aqueuse de bicarbonate de sodium, puis à l'eau. On sèche la phase organique sur du sulfate de sodium et on évapore le solvant sous pression réduite. On obtient 40 g du composé du titre.

**15b/ *Acide α,α-diméthyl-4-biphénylylacétique.***

On dissout 10,8 g (0,0449 mole) du produit de l'étape précédente dans 100 ml de diméthylformamide. On refroidit le mélange à 0-5°C et on y ajoute par petites portions, 5,04 g d'hydrure de sodium à 55%. On agite à la température ambiante pendant 30 minutes. On refroidit à nouveau à 0-5°C et on ajoute goutte à goutte 7,9 ml d'iodure de méthyle et on agite à la température ambiante pendant 3 heures. On verse dans un mélange eau/glace, on extrait à l'acétate d'éthyle, on lave à l'eau, on sèche la phase organique sur du sulfate de sodium et on évapore le solvant sous pression réduite. On dissout l'huile ainsi obtenue dans une solution de 12 g d'hydroxyde de sodium dans 70 ml d'eau et 70 ml d'éthanol à 95%. On chauffe au reflux pendant 2 heures, on évapore l'éthanol sous pression réduite, on lave la phase aqueuse à l'acétate d'éthyle. On acidifie la solution aqueuse à l'aide d'acide chlorhydrique concentré et on filtre le précipité formé. On obtient 8,2 g du composé du titre qu'on cristallise dans 500 ml d'éthanol à 50%. P.f. 152-157°C.

**15c/ *1-[2-(4-biphénylyl)-2-méthylpropionyl]-4-hydroxy-4-(3-trifluorométhylphényl)-1,2,3,6-tétrahydropyridine.***

On mélange 3,6 g (0,015 mole) du produit de l'étape précédente, 60 ml de chlorure de méthylène, 6,3 ml (0,045 mole) de triéthylamine, 4,2 g (0,015 mole) de chlorhydrate de 4-(3-trifluorométhylphényl)-4-pipéridinol et 6,6 g

(0,015 mole) de BOP et on agite à la température ambiante pendant 1,5 heures. On évapore le solvant sous pression réduite, on reprend le résidu à l'acétate d'éthyle, on lave à l'eau, puis avec une solution d'acide chlorhydrique 1N, à l'eau, avec une solution d'hydroxyde de sodium 1N et encore à l'eau. On sèche la phase organique sur du sulfate de sodium et on évapore le solvant sous pression réduite. On obtient une huile qu'on reprend à l'acétate d'éthyle. On ajoute à la solution du gel de silice et on agite pendant 15 minutes. On filtre et on évapore le solvant sous pression réduite. On obtient 6,3 g du composé du titre.

**15d/ *1-[2-(4-biphénylyl)-2-méthylpropyl]-4-hydroxy-4-(3-trifluorométhylphényl)pipéridine.***

On dissout le produit de l'étape précédente dans 70 ml de tétrahydrofuranne anhydre. On chauffe au reflux et on ajoute, goutte à goutte, une solution de 3,7 ml (0,039 mole) de boranediméthylsulfure dans 45 ml de tétrahydrofuranne anhydre. On chauffe le mélange au reflux pendant 4 heures puis on refroidit à 10-15°C et on y ajoute avec précaution, 15 ml de méthanol. On agite pendant 15 min à la température ambiante puis 30 min au reflux. On évapore le solvant sous pression réduite, on reprend le résidu dans 15 ml d'eau, on y ajoute de l'hydroxyde d'ammonium jusqu'à pH basique et on extrait à l'acétate d'éthyle. On lave la phase organique à l'eau, on sèche sur du sulfate de sodium et on évapore le solvant sous pression réduite. On obtient le composé du titre qu'on cristallise dans 50 ml de méthanol. P.f. 270-272°C.

***15e/ Chlorhydrate de 1-[2-(4-biphénylyl)-2-méthylpropyl]-4-(3-trifluorométhylphényl)-1,2,3,6-tétrahydropyridine.***

On mélange 1,7g (0,0035 mole) du produit de l'étape précédente, 14,6 ml d'acide acétique glacial et 4,4 ml d'acide sulfurique à 96%. On chauffe au reflux pendant deux heures puis on verse dans un mélange eau/glace. On ajoute de l'hydroxyde de sodium jusqu'à pH basique, on extrait à l'acétate d'éthyle, on sèche sur du sulfate de sodium et on évapore le solvant sous pression réduite. On prépare le chlorhydrate à l'aide d'isopropanol saturé en acide chlorhydrique. P.f. 238-240°C.

EXEMPLE 16

**Chlorhydrate de 1-[2-(4-phénoxyphényl)-2-éthyl]-4-(3-trifluorométhylphényl)-1,2,3,6-tétrahydropyridine.**

**16a/ *1-bromo-2-(4-phénoxyphényl)éthane.***

On refroidit à 0-5°C un mélange de 3,31 g (0,038 mole) de bromoacétylbromure, 50 ml de chlorure de méthylène, 5 g (0,029 mole) de diphényléther et on y ajoute 4,42 ml (0,033 mole) de chlorure d'aluminium. On agite le mélange à la température ambiante pendant 4 heures. On verse dans un mélange eau/glace, on sépare les deux phases, on lave la phase organique à l'acide chlorhydrique 1N et à l'eau. On sèche la phase organique sur du sulfate de sodium et on évapore le solvant sous pression réduite. On purifie le résidu par chromatographie sur colonne de gel de silice en éluant avec un mélange cyclohexane/acétate d'éthyle = 7/3. On isole la fraction du milieu correspondant à 3,2g de 2-(4-phénoxyphényl)acétylbromure. On mélange l'huile ainsi obtenue avec 5,9 ml (0,077 mole) d'acide trifluoroacétique et 7,7 ml (0,048 mole) de triéthylsilane. On chauffe à 80°C pendant 4 heures, on reprend le résidu dans un mélange éther éthylique/bicarbonate de sodium. On sépare les deux phases, on sèche la phase organique sur du sulfate de sodium et on évapore le solvant sous pression réduite. On obtient 7 g du composé du titre.

**16b/ *Chlorhydrate de 1-[2-(4-phénoxyphényl)-2-éthyl]-4-(3-trifluorométhylphényl)-1,2,3,6-tétrahydropyridine.***

On chauffe au reflux pendant 5 heures un mélange de 2,63 g (0,010 mole) de 4-(3-trifluorométhylphényl)-1,2,3,6-tétrahydropyridine, 80 ml de butanol, 3,5 g (0,025 mole) de carbonate de potassium anhydre rapé et 3,05 g (0,011 mole) du produit de l'étape précédente. On élimine les sels par filtration, on évapore le solvant sous pression réduite, on reprend à l'acétate d'éthyle, on lave à l'eau, on sèche sur du sulfate de sodium et on évapore le solvant sous pression réduite. On purifie le produit brut par chromatographie sur colonne de gel de silice en éluant avec un mélange acétate d'éthyle/cyclohexane = 3/7. On isole le produit ayant un Rf ≈ 0,5. On prépare le chlorhydrate à l'aide d'isopropanol saturé en acide chlorhydrique. P.f. 196-198°C.

EXEMPLE 17

**Chlorhydrate de 1-[2-(4-benzylphényl)-2-éthyl]-4-(3-trifluorométhylphényl)-1,2,3,6-tétrahydropyridine.**

**17a/ *1-bromo-2-(4-benzylphényl)éthane.***

On refroidit à 0-5°C un mélange de 2,7 g (0,030 mole) de bromoacétylbromure, 54 ml de chlorure de méthylène, 4 g (0,0238 mole) de diphénylméthane et on y ajoute 3,7 ml (0,0274 mole) de chlorure d'aluminium. On agite le mélange à la température ambiante pendant 4 heures. On verse dans un mélange eau/glace, on sépare les deux phases. On sèche la phase organique sur du sulfate de sodium et on évapore le solvant sous pression réduite. On reprend le résidu dans de l'éther isopropylique, on agite et on filtre le précipité formé. On isole 4,5 g de 2-(4-benzylphényl)acétylbromure. On mélange le produit ainsi obtenu avec 8 ml d'acide trifluoroacétique et 4,57 ml (0,048 mole) de triéthylsilane. On chauffe à 80°C pendant 4 heures, on reprend le résidu dans un mélange éther éthylique/bicarbonate de sodium. On sépare les deux phases, on sèche la phase organique sur du sulfate de sodium et on évapore le solvant sous pression réduite. On obtient 6,5 g du composé du titre qu'on purifie par chromatographie sur colonne de gel de silice en éluant avec un mélange cyclohexane/acétate d'éthyle = 9/1. On isole la première fraction éluée qui correspond au composé du titre.

**17b/ *Chlorhydrate de 1-[2-(4-benzylphényl)-2-éthyl]-4-(3-trifluorométhylphényl)-1,2,3,6-tétrahydropyridine.***

On chauffe au reflux pendant 5 heures un mélange de 2,6 g (0,0098 mole) de 4-(3-trifluorométhylphényl)-1,2,3,6-tétrahydropyridine, 80 ml de butanol, 3,4 g (0,0246 mole) de carbonate de potassium anhydre râpé et 3 g du produit de l'étape précédente. On élimine les sels par filtration, on évapore le solvant sous pression réduite, on reprend à l'acétate d'éthyle, on lave à l'eau, on sèche sur du sulfate de sodium et on évapore le solvant sous pression réduite. On purifie le produit brut par chromatographie sur colonne de gel de silice en éluant avec un mélange acétate d'éthyle/cyclohexane = 3/7. On isole 2 g de base. On prépare le chlorhydrate à l'aide d'isopropanol saturé en acide chlorhydrique. On obtient le composé du titre qu'on cristallise dans de l'acétone. P.f. 169-172°C.

EXEMPLE 18

**Oxalate de 1-[2-(4-n-butylphényl)éthyl]-4-(3-trifluorométhylphényl)-1,2,3,6-tétrahydropyridine.**

**18a/*1-bromo-2-(4-n-butylphényl)éthane.***

En opérant comme décrit dans l'exemple 8a, mais en utilisant 4,7 ml (0,030 mole) de n-butylbenzène au lieu du 3-chlorobiphényle, on obtient 2,5 g du composé du titre. Chromatographie sur couche mince (éluant cyclohexane): R.f. 0,4.

**18b/*Oxalate de 1-[2-(4-n-butylphényl)éthyl]-4-(3-trifluorométhylphényl)-1,2,3,6-tétrahydropyridine.***

En opérant comme décrit dans l'exemple 8b, mais en utilisant le composé de l'étape 18a, on obtient la 1-[2-(4-n-butylphényl)éthyl]-4-(3-trifluorométhylphényl)-1,2,3,6-tétrahydropyridine brute qu'on purifie par chromatographie sur colonne de gel de silice en éluant avec un mélange acétate d'éthyle/cyclohexane = 1/1. On prépare l'oxalate à l'aide d'acide oxalique dans de l'acétone. On cristallise le produit ainsi obtenu dans de l'isopropanol. On obtient 1,18 g du composé du titre. P.f. 162-165°C.

EXEMPLE 19

**1-[2-(4-biphénylyl)éthyl]-4-(3-trifluorométhylphényl)-1,2,3,6-tétrahydropyridine et son chlorhydrate.**

**19a/ *Ester éthylique de l'acide 4-biphénylylacétique.***

En opérant comme décrit dans l'exemple 1a, en utilisant l'acide 4-biphénylylacétique au lieu de l'acide 2-(4-isobutylphényl)propionique, on obtient le composé du titre.

**19b/ *Alcool 4-biphénylyléthylique.***

En opérant comme décrit dans l'exemple 1b, en utilisant le produit de l'étape 19a, on obtient le composé du

titre. P.f. 75-80°C.

**19c/ *2-(4-biphénylyl)éthyl méthanesulfonate.***

En opérant comme décrit dans l'exemple 1c, en utilisant le produit de l'étape 19b, on obtient le composé du titre. P.f. 75-77°C.

**19d/ *1-[2-(4-biphénylyl)éthyl]-4-(3-trifluorométhylphényl)-1,2,3,6-tétrahydropyridine et son chlorhydrate.***

On chauffe au reflux pendant 8 heures un mélange de 8,3 g (0,03 mole) du produit de l'étape précédente, 100 ml d'isopropanol, 12,8 ml (0,0915 mole) de triéthylamine et 7,91 g (0,03 mole) de 4-(3-trifluorométhylphényl)-1,2,3,6-tétrahydropyridine. On évapore le solvant sous pression réduite et on reprend le résidu dans 70 ml d'acétate d'éthyle. On lave 2 fois à l'eau, on sèche sur du sulfate de sodium et on évapore le solvant sous pression réduite. On obtient la 1-[2-(4-biphénylyl)éthyl]-4-(3-trifluorométhylphényl)-1,2,3,6-tétrahydropyridine qu'on cristallise dans 30 ml d'isopropanol. P.f. 114-116°C. On prépare le chlorhydrate à l'aide d'isopropanol saturé en acide chlorhydrique. On obtient le composé du titre qu'on cristallise dans de l'éthanol à 95%. P.f. 262-263°C.

EXEMPLE 20

**Chlorhydrate de 1-[2-(4-n-butoxyphényl)éthyl]-4-(3-trifluorométhylphényl)-1,2,3,6-tétrahydropyridine.**

**20a/ *1-[2-(4-hydroxyphényl)éthyl]-4-(3-trifluorométhylphényl)-1,2,3,6-tétrahydropyridine et son chlorhydrate.***

On chauffe au reflux pendant 5 heures un mélange de 5,6 g (0,0211 mole) de 4-(3-trifluorométhylphényl)-1,2,3,6-tétrahydropyridine, 7,3 g (0,0528 mole) de carbonate de potassium, 112 ml d'alcool amylique et 3,3 g (0,0211 mole) de 4-hydroxyphénéthylchlorure. On filtre les sels éventuels, on évapore le solvant sous pression réduite, on reprend à l'acétate d'éthyle, on lave à l'eau, on sèche la phase organique sur du sulfate de sodium et on évapore le solvant sous pression réduite. On prépare le chlorhydrate à l'aide d'isopropanol saturé en acide chlorhydrique. On obtient 3 g du composé du titre. P.f. 220-222°C.

**20b/ *Chlorhydrate de 1-[2-(4-n-butoxyphényl)éthyl]-4-(3-trifluorométhylphényl)-1,2,3,6-tétrahydropyridine.***

On agite pendant 2 heures à la température ambiante un mélange de 1 g (0,0029 mole) du composé de l'étape précédente sous forme de base, 15 ml de DMSO, 150 mg (0,0038 mole) d'hydrure de sodium à 60%. On ajoute au mélange réactionnel 0,4 ml (0,0038 mole) de 1-bromobutane et 150 mg (0,001 mole) de iodure de potassium. On agite encore à la température ambiante pendant deux heures. On verse dans un mélange eau/glace, on extrait à l'acétate d'éthyle, on sèche la phase organique sur du sulfate de sodium et on évapore le solvant sous pression réduite. On prépare le chlorhydrate à l'aide d'isopropanol saturé en acide chlorhydrique. On obtient 500 mg du composé du titre. P.f. 212-214°C.

EXEMPLE 21

**Chlorhydrate de 1-[2-(4-(3-éthoxycarbonylpropoxy)phényl)éthyl]-4-(3-trifluorométhylphényl)-1,2,3,6-tétrahydropyridine.**

On opère comme décrit dans l'exemple 20b, en utilisant l'ester éthylique de l'acide 4-bromobutyrique au lieu du 1-bromobutane. On obtient le composé du titre qu'on cristallise dans de l'isopropanol. P.f. 204-205°C.

| Analyse élémentaire | | % C | % H | % N |
|---|---|---|---|---|
| | calculé | 62,71 | 6,27 | 2,81 |
| | trouvé | 62,69 | 6,34 | 2,80 |

EXEMPLE 22

**1-[2-(4-biphénylyl)éthyl]-4-(6-chloropyrid-2-yl)-1,2,3,6-tétrahydropyridine. 22a/ *(1-benzyl-1,2,3,6-tétrahydropyrid-4-yl)tributylstannane.***

On agite à la température ambiante pendant 3 heures un mélange de 15,85 g (0,0837 mole) de 1-benzyl-4-pipéridone dans 140 ml de diméthoxyéthane anhydre et 25 g (0,0837 mole) de trisilidrazine dans 140 ml de diméthoxyéthane anhydre. On évapore le solvant sous pression réduite. On reprend le résidu dans 420 ml d'hexane anhydre et on y ajoute 420 ml de tétraméthyléthylènediamine anhydre. On refroidit le mélange à -78°C et on y ajoute goutte à goutte 156 ml de n-butyl lithium (0,25 mole) (solution 1,6 M dans l'hexane). Après environ 30 minutes on laisse revenir la température jusqu'à 0°C et on agite pendant 15 minutes. On ajoute ensuite au mélange réactionnel 45 ml (0,167 mole) de chlorure de tributylstannane. Après 1 heure on ajoute, avec extrème précaution un mélange eau/glace. On extrait à l'éther éthylique, on lave la phase organique à l'eau, on sèche sur du sulfate de sodium et on évapore le solvant sous pression réduite. On obtient 70 g de produit brut qu'on purifie par chromatographie sur colonne de gel de silice en éluant avec un mélange cyclohexane/acétate d'éthyle = 95/5. On obtient le composé du titre sous forme d'huile.

$^1$H-RMN ($CDCl_3$) - d (ppm) : 0,84 (9H; m: $CH_3$); 1,19-1,58 (18H; m: $CH_2$ - chaîne); 2,31 (2H; m); 2,53 (2H; m); 3,02 (2H; m); 3,56 (2H; s: méthylène benzylique); 5,76 (1H; m*); 7,14-7,18 (5H; m: arom.)

* bandes satellites $^3J_{cis}(^1H-^{117}Sn)$ et $^3J_{cis}(^1H-^{119}Sn)$.

**22b/ *1-benzyl-4-(6-chloropyrid-2-yl)-1,2,3,6-tétrahydropyridine.***

On dissout 18,5 g (0,04 mole) du composé de l'étape précédente dans 200 ml de diméthylformamide anhydre en atmosphère d'azote. On ajoute à la solution 11,8 g (0,08 mole) de 2,6-dichloropyridine, 0,64 g de Pd(II) $(Ph_3P)_2Cl_2$, 4,38 g (0,04 mole) de chlorure de tétraméthylammonium et 2,76 g (0,02 mole) de carbonate de potassium. On chauffe à 110°C pendant 6 heures puis on verse le mélange dans 100 ml d'une solution d'acide sulfurique à 5%. On extrait à l'éther éthylique, on ajoute de l'hydroxyde d'ammonium à la phase aqueuse jusqu'à pH basique et on extrait à l'acétate d'éthyle. On sèche les phases organiques réunies sur du sulfate de sodium et on évapore le solvant sous pression réduite. On purifie le résidu par chromatographie sur colonne de gel de silice en éluant avec un mélange cyclohexane/acétate d'éthyle = 1/1. On obtient le composé du titre. P.f. 100-102°C.

**22c/ *Chlorhydrate de 4-(6-chloropyrid-2-yl)-1,2,3,6-tétrahydropyridine.***

On refroidit à 0-5°C une solution de 7,0 g (0,024 mole) du composé de l'étape précédente dans 110 ml de dichloroéthane et on y ajoute 5,8 ml (0,054 mole) de chloroéthylchloroformiate. On agite pendant 5 minutes puis on chauffe au reflux pendant 1,5 heures. On évapore le solvant sous pression réduite, on reprend le résidu dans 100 ml de méthanol et on chauffe au reflux pendant 1 heure. On évapore le solvant, on reprend le résidu dans de l'isopropanol et on filtre le solide. On obtient le composé du titre qu'on cristallise dans de l'éthanol à 90%. P.f. 305-307°C.

**22d/ *1-[2-(4-biphénylyl)éthyl]-4-(6-chloropyrid-2-yl)-1,2,3,6-tétrahydropyridine.***

On chauffe au reflux pendant 6 heures un mélange de 1,35 g (0,0053 mole) de 4-(2-bromoéthyl)biphényle, 25 ml de butanol, 1,72 g (0,0125 mole) de carbonate de potassium anhydre rapé et 1,16 g (0,005 mole) du produit de l'étape précédente. On évapore le solvant sous pression réduite, on reprend le résidu à l'acétate d'éthyle, on lave à l'eau, on sèche sur du sulfate de sodium et on évapore le solvant sous pression réduite. On reprend le résidu dans 30 ml d'éther isopropylique et on filtre le précipité formé. On obtient le composé du titre qu'on recristallise dans de l'isopropanol. P.f. 135-136°C.

EXEMPLE 23

**Chlorhydrate de 1-[2-(2,3'-dichloro-4-biphenylyl)éthyl]-4-(3-trifluorométhylphényl)-1,2,3,6,-tétrahydropyridine.**

**23a/*Ester éthylique de l'acide (3-chloro-4-trifluorométhylsulfonylphényl)acétique.***

Dans une solution de 5 g (27 mmole) d'acide (3-chloro-4-hydroxyphényl)-acétique dans 60 ml d'éthanol on fait barboter de l'acide chlorhydrique gazeux, en refroidissant dans un bain de glace pendant une heure, puis on chauffe au reflux pendant 5 heures. On évapore le solvant, on reprend le résidu avec une solution aqueuse saturée de bicarbonate de sodium et on extrait à l'acétate d'éthyle. On filtre, on sèche la phase organique sur du sulfate de sodium et

on évapore le solvant sous pression réduite. On purifie le produit brut par chromatographie sur colonne de gel de silice en éluant avec un mélange cyclohexane/acétate d'éthyle = 7/3, en obtenant l'ester éthylique de l'acide 3-chloro-4-hydroxyphénylacétique.

On dissout 4 g (18,6 mmole) de ce composé dans 14 ml de pyridine et on y ajoute goutte à goutte, sous atmosphère d'azote, 3,36 ml (20 mmole) d'anhydride triflique, tout en maintenant la température à 0°C pendant 1 heure. On verse le mélange dans de la glace et on extrait à l'acétate d'éthyle. On sèche la phase organique sur du sulfate de sodium, on filtre et on évapore le solvant sous pression réduite. On purifie le brut par chromatographie sur colonne de gel de silice, en éluant avec un mélange cyclohexane/acétate d'éthyle = 9/1. On obtient le composé du titre.

**23b/** ***Ester éthylique de l'acide (2,3'-dichloro-4-biphénylyl)acétique.***

On agite à 70°C pendant 1 heure, un mélange de 4,9 g (14 mmole) du produit de l'étape précédente, 2,45 g (16 mmole) d'acide 3-chlorobenzèneboronique, 63 mg (0,28 mmole) d'acétate de palladium, 4,84 g (35 mmole) de carbonate de potassium et 4,5 g (14 mmole) de bromure de tétrabutylammonium dans 19 ml d'eau. On laisse refroidir et on extrait à l'acétate d'éthyle. On sèche la phase organique sur du sulfate de sodium, on filtre et on évapore le solvant sous pression réduite. On purifie le brut de réaction par chromatographie sur colonne de gel de silice, en éluant avec un mélange cyclohexane/acétate d'éthyle = 9/1 On obtient le composé du titre sous forme huileuse.

**23c/*****Acide (2,3'-dichloro-4-biphénylyl)acétique.***

On chauffe à 80°C pendant 2 heures, un mélange du produit obtenu à l'étape précédente et de 1,57 g (28 mmole) d'hydroxyde de potassium dans 39 ml de méthanol. On évapore le solvant sous pression réduite, on lave le résidu avec une solution 1N d'acide chlorhydrique et on extrait au chlorure de méthylène. On sèche la phase organique sur du sulfate de sodium, on filtre et on évapore le solvant sous pression réduite. On traite le produit brut avec de l'hexane et on obtient un précipité blanc qu'on filtre et cristallise dans un mélange hexane/acétate d'éthyle. On obtient 1,4 g du produit du titre.P.f.92-94°C.

**23d/** ***1-[(2,3'-dichloro-4-biphénylyl)acétyl]-4-hydroxy-4-(3-trifluorométhylphényl)pipéridine.***

On agite à la température ambiante pendant 24 heures, un mélange de 1,2 g (4,3 mmole) du produit de l'étape précédente, 1,2 g (4,3 mmole) de 4-hydroxy-4-(3-trifluorométhylphényl)pipéridine, 17,2 ml de chlorure de méthylène, 1,54 ml (11 mmole) de triéthylamine et 1,9 g (4,3 mmole) de BOP. On verse dans l'eau, on extrait au chlorure de méthylène, on lave la phase organique avec une solution 1N d'acide chlorhydrique, avec de l'eau, avec une solution 1N d'hydroxyde de sodium et encore avec de l'eau. On sèche la phase organique sur du sulfate de sodium, on filtre et on évapore le solvant sous pression réduite. On purifie le produit brut par chromatographie sur colonne de gel de silice, en éluant avec un mélange cyclohexane /acétate d'éthyle = 1/1. On obtient le produit du titre sous forme de solide blanc.P.f. 154-155°C.

**23e/** ***1-[2-(2,3'-dichloro-4-biphénylyl)éthyl]-4-hydroxy-4-(3-trifluorométhylphényl)pipéridine.***

On chauffe au reflux 1,6 g (3,15 mmole) du composé de l'étape précédente dans 17 ml de tétrahydrofuranne et on y ajoute 0,92 ml (9,3 mmole) de boranediméthylsulfure dans 12 ml de tétrahydrofuranne en maintenant le reflux 4 heures. On refroidit la solution à 0°C, on y ajoute 15 ml de méthanol, on agite pendant 30 minutes au reflux. On évapore le solvant sous pression réduite, on reprend le résidu à l'eau (15 ml), on y ajoute de l'hydroxyde d'ammonium concentré jusqu'à pH basique, on extrait à l'acétate d'éthyle, on lave à l'eau, on sèche la phase organique sur du sulfate de sodium et on évapore le solvant sous pression réduite. On purifie le produit par chromatographie sur colonne de gel de silice, en éluant avec un mélange cyclohexane/acétate d'éthyle = 1/1. On obtient 1,2 g du produit du titre sous forme huileuse.

**23f/** ***Chlorhydrate de 1-[2-(2,3'-dichloro-4-biphénylyl)éthyl]-4-(3-trifluorométhylphényl)-1,2,3,6-tétrahydropyridine.***

On chauffe à 100°C pendant 1 heure 1,2 g (2,43 mmole) du composé de l'étape précédente, 12,7 ml d'acide acétique glacial, 3 ml d'acide sulfurique à 96%. On verse dans un mélange eau/glace, on y ajoute une solution d'hydroxyde de sodium à 20% jusqu'à pH basique, on extrait à l'acétate d'éthyle, on lave la phase organique à l'eau, on la sèche sur du sulfate de sodium et on évapore le solvant sous pression réduite. On purifie le produit par chromatographie sur colonne de gel de silice en éluant avec un mélange cyclohexane/acétate d'éthyle = 7/3. On prépare le chlorhydrate du produit obtenu à l'aide d'une solution d'isopropanol saturée en acide chlorhydrique.

On obtient un solide blanc. P.f. 204-206°C.

EXEMPLE 24

**Chlorhydrate de 1-[2-(3-chloro-4-biphénylyl)éthyl]-4-(3-trifluorométhylphényl)-1,2,3,6-tétrahydropyridine.**

**24a/ *Ester éthylique de l'acide (2-chloro-4-trifluorométhylsulfonylphényl)-acétique***

En opérant comme décrit dans l'exemple 23a mais en utilisant l'acide (2-chloro-4-hydroxyphényl)acétique préparé selon la préparation 4, au lieu de l'acide (3-chloro-4-hydroxyphényl)acétique, on obtient le composé du titre.

**24b/ *Ester éthylique de l'acide (3-chloro-4-biphénylyl)acétique***

En opérant comme décrit dans l'exemple 23b mais en utilisant le produit de l'étape précédente au lieu du produit de l'étape 23a et l'acide benzèneboronique au lieu de l'acide 3-chlorobenzèneboronique, on obtient le composé du titre.

**24c/*Acide (3-chloro-4-biphénylyl)acétique***

En opérant comme décrit dans l'exemple 23c mais en utilisant l'ester de l'étape précédente, on obtient le composé du titre.

**24d/ *1-[(3-chloro-4-biphénylyl)acétyl]-4-hydroxy-4-(3-trifluorométhylphényl)pipéridine.***

En opérant comme décrit dans l'exemple 23d mais en utilisant le produit de l'étape précédente au lieu du produit de l'étape 23c, on obtient le composé du titre.

**24e/ *1-[2-(3-chloro-4-biphénylyl)éthyl]-4-hydroxy-4-(3-trifluorométhylphényl)pipéridine.***

En opérant comme décrit dans l'exemple 23e mais en utilisant le produit de l'étape précédente au lieu du produit de l'étape 23d on obtient le composé du titre.

**24f/ Chlorhydrate de *1-[2-(3-chloro-4-biphénylyl)éthylJ-4-(3-trifluorométhylphényl)-1,2,3,6-tétrahydropyridine***

En opérant comme décrit dans l'exemple 23f mais en utilisant le produit de l'étape précédente au lieu du produit de l'étape 23e on obtient le composé du titre. P.f. 227°C.

EXEMPLE 25

**Chlorhydrate de 1-[2-(3',5'-dichloro-4-biphénylyl)éthyl]-4-(3-trifluorométhylphényl)-1,2,3,6-tétrahydropyridine.**

**25a/ *2-(3',5'-dichloro-4-biphénylyl)éthanol.***

En opérant comme décrit dans l'exemple 23b mais en utilisant du 2-(p-bromophényl)éthanol au lieu de l'ester éthylique de l'acide (3-chloro-4-trifluorométhylsulfonylphényl)acétique et l'acide 3,5-dichlorobenzèneboronique au lieu de l'acide 3-chlorobenzèneboronique, on obtient le composé du titre.

**25b/ *2-(3',5'-dichloro-4-biphénylyl)éthyl méthanesulfonate.***

En opérant comme décrit dans l'exemple 1c mais en utilisant le produit de l'étape précédente au lieu de l'alcool 2-(4-isobutylphényl)propylique on obtient le composé du titre.

**25c/ *Chlorhydrate de 1-[2-(3',5'-dichloro-4-biphénylyl)éthyl]-4-(3-trifluorométhylphényl)-1,2,3,6-tétrahydropyridine.***

En opérant comme décrit dans l'exemple 1d mais en utilisant le produit de l'étape précédente au lieu du 2-(4-isobutylphényl)propyl méthanesulfonate, on obtient le composé du titre. P.f.200-202°C.

EXEMPLE 26

**Chlorhydrate de 1-[2-(2',4'-dichloro-4-biphénylyl)éthyl]-4-(3-trifluorométhylphényl)-1,2,3,6-tétrahydropyridine.**

En opérant comme décrit dans l'exemple 25 mais en utilisant l'acide 2,4-dichlorobenzèneboronique au lieu de l'acide 3,5-dichlorobenzèneboronique, on obtient le composé du titre. P.f. 204-206°C.

EXEMPLE 27

**Chlorhydrate de 1-[2-(2-chloro-4-biphénylyl)éthyl]-4-(3-trifluorométhylphényl)-1,2,3,6-tétrahydropyridine.**

**27a/ *Acide (2-chloro-4-biphénylyl)acétique.***

En opérant comme décrit dans l'exemple 23c mais en utilisant l'acide benzèneboronique au lieu de l'acide 3-chlorobenzèneboronique, on obtient le composé du titre sous forme de solide qu'on cristallise dans de l'acétate d'éthyle. P.f. 103-105°C.

**27b/ *1-[(2-chloro-4-biphénylyl)acétyl]-4-hydroxy-4-(3-trifluorométhylphényl)pipéridine.***

En opérant comme décrit dans l'exemple 23d mais en utilisant le composé de l'étape précédente au lieu de l'acide (2,3'-dichloro-4-biphénylyl)acétique, on obtient le composé du titre. P.f. 46-49°C.

**27c/ *Chlorhydrate de 1-[2-(2-chloro-4-biphénylyl)éthyl]-4-(3-trifluorométhylphényl)-1,2,3,6-tétrahydropyridine.***

En opérant comme décrit dans l'exemple 23e et 23f mais en utilisant le produit de l'étape précédente au lieu de la 1-[(2,3'-dichloro-4-biphénylyl)acétyl]-4-hydroxy-4-(3-trifluorométhylphényl)pipéridine, on obtient le composé du titre. P.f. 210-212°C.

EXEMPLE 28

**Chlorhydrate de 1-[2-(3'-chloro-4-biphénylyl)]-2-méthylpropyl]-4-(3-trifluorométhylphényl)-1,2,3,6-tétrahydropyridine.**

**28a/ *Ester éthylique de l'acide 2-(4-bromophényl)-2-méthylpropionique.***

On dissout 7,5 g (31 mmole) d'ester éthylique de l'acide 4-bromophénylacétique dans 120 ml de DMF et on y ajoute lentement 2,5 g d'hydrure de sodium (dispersion à 60 % en huile). On agite le mélange à la température ambiante pendant 30 minutes puis on refroidit à 10°C et on y ajoute 4,1 ml (61 mmole) de iodure de méthyle et on agite à la température ambiante pendant 4 heures. On verse dans un mélange eau/glace, on extrait à l'acétate d'éthyle, on sèche la phase organique sur du sulfate de sodium, on filtre et on évapore le solvant sous pression réduite. On purifie le brut par chromatographie sur colonne de gel de silice, en éluant avec un mélange cyclohexane/acétate d'éthyle = 95/5. On obtient le composé du titre.

**28b/ *Acide 2-(3'-chloro-4-biphénylyl)-2-méthylpropionique.***

On chauffe à 70°C pendant 3 heures, sous atmosphère d'argon, un mélange de 2,18 g (8 mmole) du produit de l'étape précédente, 1,38 g (8,8 mmole) d'acide 3-chlorobenzèneboronique, 2,76 g (20 mmole) de bicarbonate de potassium, 2,58 g (8 mmole) de bromure de tétrabutylammonium et 40 mg d'acétate de palladium dans 11 ml d'eau. Après refroidissement, on extrait à l'acétate d'éthyle. On sèche la phase organique sur du sulfate de sodium, on filtre et on évapore le solvant sous pression réduite.

On purifie le brut de réaction par chromatographie sur colonne de gel de silice en éluant avec un mélange cyclohexane/acétate d'éthyle = 9/1. On obtient 1,85 g de l'ester éthylique de l'acide du titre. On mélange ce produit avec 0,7 g d'hydroxyde de potassium dans 14 ml de méthanol et on chauffe à 80°C pendant 3 heures. On évapore le solvant et on verse le résidu dans de l'eau. On acidifie le mélange à l'aide d'une solution 1N d'acide chlorhydrique. On extrait au chlorure de méthylène, on sèche la phase organique sur du sulfate de sodium, on filtre et on évapore le solvant sous pression réduite. On cristallise le solide obtenu dans un mélange hexane/acétate d'éthyle. On obtient 1 g du composé du titre sous forme de solide blanc. P.f. 145-146°C.

**28c/ *Chlorhydrate de 1-[2-(3'-chloro-4-biphénylyl)-2-méthylpropyl]-4-(3-trifluorométhylphényl)-1,2,3,6-tétrahydropyridine.***

En opérant comme décrit dans les exemples 23d, 23e et 23f mais en utilisant le composé de l'étape précédente au lieu de l'acide (2,3'-dichloro-4-biphénylyl)-acétique, on obtient le composé du titre. P.f. 215-217°C.

EXEMPLE 29

**Chlorhydrate de 1-[2-(2-fluoro-4-biphénylyl)propyl]-4-(3-trifluorométhylphényl)-1,2,3,6-tétrahydropyridine.**

En opérant comme décrit dans les exemples 23d, 23e et 23f mais en utilisant le flurbiprofène au lieu de l'acide (2,3'-dichloro-4-biphénylyl)acétique, on obtient le composé du titre. P.f. 181-183°C (base libre); P.f. 204-206°C (chlorhydrate).

EXEMPLE 30

**Chlorhydrate de 1-[2-(4-méthoxy-3-biphénylyl)éthyl]-4-(3-trifluorométhylphényl)-1,2,3,6-tétrahydropyridine et 1-[2-(4'-méthoxy-4-biphénylyl)éthyl]-4-(3-trifluorométhylphényl)-1,2,3,6-tétrahydropyridine.**

On mélange 11,5 g (0,0652 mole) de 4-méthoxybiphényle, 50 ml de dichloroéthane et 5,42 g (0,0625 mole) de bromoacétylbromure. On refroidit à - 10°C et on ajoute 9,3 g (0,070 mole) de trichlorure d'aluminium. On agite à - 10°C pendant 2 heures, on acidifie à l'aide d'une solution 1N d'acide chlorhydrique, on sépare les deux phases et on extrait la phase aqueuse à l'acétate d'éthyle. On sèche les phases organiques réunies sur du sulfate de sodium, on évapore le solvant sous pression réduite et on purifie le brut par chromatographie sur colonne de gel de silice, en éluant avec un mélange cyclohexane/acétate d'éthyle = 9/1. On isole une fraction à Rf = 0,3 constituée par un mélange 75 : 25 des isomères 3-bromoacétyl-4-méthoxybiphényle et 4'-bromoacétyl-4-méthoxybiphényle. On chauffe au reflux pendant 3 heures 5,8 g (0,019 mole) du mélange d'isomères ci-dessus dans 11,6 ml d'acide trifluoroacétique et 5,8 ml de triéthylsilane. On verse dans de la glace, on y ajoute une solution 1N d'hydroxyde de sodium jusqu'à pH basique et on extrait à l'acétate d'éthyle. On sèche la phase organique sur du sulfate de sodium et on évapore le solvant sous pression réduite. On reprend le résidu par 160 ml de butanol et on y ajoute 5,1 g (0,019 mole) de 4-(3-trifluorométhylphényl)-1,2,3,6-tétrahydropyridine et 5,9 g (0,0427 mole) de carbonate de potassium. On chauffe au reflux pendant 6 heures, on filtre les sels formés et on évapore le solvant sous pression réduite. On reprend le résidu par de l'acétate d'éthyle, on lave à l'eau, on sèche la phase organique sur du sulfate de sodium et on évapore le solvant sous pression réduite. On purifie par chromatographie sur colonne de gel de silice, en éluant par un mélange cyclohexane/acétate d'éthyle = 7/3. On isole la fraction à Rf - 0,5 (chromatographie sur couche mince, éluant cyclohexane/acétate d'éthyle = 1/1). Le produit ayant le Rf légèrement supérieur correspond à la 1-[2-(4-méthoxy-3-biphénylyl)éthyl]-4-(3-trifluorométhylphényl)-1,2,3,6-tétrahydropyridine dont on prépare le chlorhydrate à l'aide d'une solution d'éther éthylique saturée en acide chlorhydrique. On obtient 0,9 g. P.f. 185°-187°C (cristallisé dans l'acétone).

Le produit ayant le Rf legèrement inférieur correspondant à la 1-[2-(4'-méthoxy-4-biphénylyl)éthyl]-4-(3-trifluorométhylphényl)-1,2,3,6-tétrahydropyridine est isolé sous forme solide dans l'éther isopropylique. P.f. 120-123°C.

EXEMPLE 31

**Chlorhydrate de 1-[2-(4'-hydroxy-4-biphénylyl)éthyl]-4-(3-trifluorométhylphényl)-1,2,3,6-tétrahydropyridine.**

On dissout 0,5 g (1,14 mmole) de 1-[2-(4'-méthoxy-4-biphénylyl)éthyl]-4-(3-trifluorométhylphényl)-1,2,3,6-tétrahydropyridine préparée selon l'exemple 30, dans 3,5 ml d'acide bromhydrique à 33 % dans l'acide acétique et on chauffe au reflux pendant 3,5 heures. On verse dans de la glace, on ajoute au mélange une solution concentrée d'hydroxyde d'ammonium et on extrait à l'acétate d'éthyle. On lave la phase organique à l'eau, on sèche et on évapore le solvant sous pression réduite. On purifie par chromatographie sur colonne de gel de silice, en éluant par un mélange cyclohexane/acétate d'éthyle = 7/3. On prépare le chlorhydrate à l'acide d'une solution d'éther éthylique saturée en acide chlorhydrique. On obtient 0,43 g du produit du titre qu'on cristallise dans de l'éthanol à 95 %. P.f. 248°-254°C.

EXEMPLE 32

**Chlorhydrate de 1-[2-(4'-éthoxycarbonylbutoxy-4-biphénylyl)éthyl]-4-(3-triflorométhylphényl)-1,2,3,6-tétrahydropyridine.**

On agite à la température ambiante pendant 2 heures un mélange de 400 mg (0,9 mmole) du produit de l'exemple 31 (base libre), 5 ml de DMSO et 49 mg d'hydrure de sodium à 60%. On y ajoute 46 mg de iodure de potassium et 0,17 ml (1 mmole) d'ester éthylique de l'acide 1-bromobutyrique. On agite à la température ambiante pendant 2 heures, on verse dans de l'eau et on extrait à l'acétate d'éthyle. On lave la phase organique à l'eau, on sèche sur du sulfate de sodium et on évapore le solvant sous pression réduite. On purifie par chromatographie sur colonne de gel de silice en éluant avec un mélange cyclohexane/acétate d'éthyle = 8/2. On prépare le chlorhydrate à l'aide d'un mélange éther éthylique/isopropanol saturé en acide chlorhydrique. On obtient le composé du titre. P.f. 242-247°C.

EXEMPLE 33

**Chlorhydrate de 1-[2-(3-biphénylyl)éthyl]-4-(3-trifluorométhylphényl)-1,2,3,6-tétrahydropyridine.**

**33a/** ***2-(3-biphénylyl)éthanol***

En opérant comme décrit dans l'exemple 23b, mais en utilisant le 3-bromophénéthylalcool au lieu de l'ester de l'acide (3-chloro-4-trifluorométhylsulfonylphényl)acétique et l'acide benzèneboronique au lieu de l'acide 3-chlorobenzèneboronique, on obtient le composé du titre. P.f. 58-60°C.

**33b/** ***2-(3-biphénylyl)éthyl-p-toluènesulfonate***

On agite à la température ambiante pendant 1 nuit 0,7 g (3,5 mmole) du produit de l'étape précédente et 1 g (5,2 mmole) de chlorure de tosyle dans 5 ml de pyridine. On verse dans une solution 1 N d'acide chlorhydrique et on extrait à l'acétate d'éthyle. On lave la phase organique avec une solution 3 M d'hydroxyde de sodium. On sèche sur du sulfate de sodium et on évapore sous pression réduite. On purifie par chromatographie sur colonne de gel de silice en éluant avec un mélange cyclohexane/acétate d'éthyle = 9/1, et ensuite 8/2. On obtient le composé du titre sous forme huileuse.

**33c/** ***Chlorhydrate de 1-[2-(3-biphénylyl)éthyl]-4-(3-trifluorométhylphényl)-1,2,3,6-tétrahydropyridine***

En opérant comme décrit dans l'exemple 1d, mais en utilisant le produit de l'étape précédente au lieu du 2-(4-isobutylphényl)propyl méthanesulfonate, on obtient le composé du titre. P.f. 191-192°C.

EXEMPLE 34

**Chlorhydrate de 1-[2-(3'chloro-4'-fluoro-4-biphénylyl)éthyl]-4-(3-trifluorométhylphényl)-1,2,3,6-tétrahydropyridine.**

**34a/** ***2-(3'-chloro-4'-fluoro-4-biphénylyl)éthanol***

En opérant comme décrit dans l'exemple 23b, mais en utilisant du 2-(p-bromophényl)éthanol au lieu de l'ester éthylique de l'acide (3-chloro-4-trifluorométhylsulfonylphényl)acétique et l'acide 3-chloro-4-fluorobenzèneboronique au lieu de l'acide 3-chlorobenzèneboronique, on obtient le composé du titre.

**34b/** ***2-(3'-chloro-4'-fluoro-4-biphénylyl)éthyl méthanesulfonate***

En opérant comme décrit dans l'exemple 1c, mais en utilisant le produit de l'étape précédente au lieu de l'alcool 2-(4-isobutylphényl)propylique, on obtient le composé du titre.

**34c/ Chlorhydrate de 1-[2-(3'-chloro-4'-fluoro-4-biphénylyl)éthyl]*-4-(3-trifluorométhylphényl)-1,2,3,6-tétrahydropyridine***

En opérant comme décrit dans l'exemple 1d, mais en utilisant le produit de l'étape précédente au lieu du

2-(4-isobutylphényl)propyl méthanesulfonate, on obtient le composé du titre. P.f. 218-220°C.

EXEMPLE 35

**Chlorhydrate de 1-[2-(2'-trifluorométhyl-4-biphénylyl)éthyl]-4-(3-trifluorométhylphényl)-1,2,3,6-tétrahydropyridine.**

**35a/** ***2-(4-bromophényl)-2,2-diméthoxyéthane***

On chauffe au reflux pendant 3 heures un mélange de 2 g (0,01 mole) de 4-bromoacétophénone, 5,6 ml de triméthylorthoformiate, 5,6 ml de méthanol et 0,67 g d'Amberlite® IR 120. Après refroidissement, on filtre sur Célite et on évapore la solution filtrée. On obtient 2,4 g du produit du titre sous forme huileuse.

**35b/** ***2,2-diméthoxy-2-(2'-trifluorométhyl-4-biphénylyl)éthane***

En opérant comme décrit dans l'exemple 23b mais en utilisant le produit de l'étape précédente au lieu de l'ester éthylique de l'acide (2,3'-dichloro-4-biphénylyl)acétique et l'acide 2-trifluorométhylphénylbenzèneboronique au lieu de l'acide 3-chlorobenzèneboronique, on obtient le composé du titre.

**35c/** ***4-(2-trifluorométhylphényl)acétophénone***

A une solution de 4,6 g (0,0105 mole) du produit de l'étape précédente dans 4 ml de chlorure de méthylène, on ajoute à 0°C une solution de 4 ml d'acide trifluoroacétique et 4 ml d'eau. On agite à la température ambiante pendant 2 heures, on verse dans de l'eau, on extrait au chlorure de méthylène. On sèche la phase organique, on filtre et on évapore le solvant sous pression réduite. On purifie le brut par chromatographie sur colonne de gel de silice en éluant avec un mélange cyclohexane/acétate d'éthyle = 9/1. On obtient 1,97 g du produit du titre.

**35d/** ***α-bromo-4-(2-trifluorométhylphényl)acétophénone***

A une solution de 1,97 g (7,5 mmole) du produit de l'étape précédente dans 5,4 ml de méthanol, on ajoute goutte à goutte à la température de 0°C, 0,38 ml (7,5 mmole) de brome. On agite à la température ambiante pendant 3 heures, on évapore le solvant, on reprend le résidu dans de l'eau et on extrait à l'acétate d'éthyle. On sèche la phase organique sur du sulfate de sodium, on filtre et on évapore le solvant sous pression réduite. On obtient 2,3 g du produit du titre.

**35e/** ***1-bromo-2-(2 '-trifluorométhyl-4-biphénylyl)éthane***

On mélange 1,2 g (3,5 mmole) du produit de l'étape précédente, 4,4 ml d'acide trifluoroacétique et 2,3 ml de triéthylsilane et on chauffe au reflux pendant 3 heures. On verse ensuite dans un mélange constitué par une solution d'hydroxyde de sodium concentrée et de la glace, on extrait à l'acétate d'éthyle, on sèche la phase organique sur du sulfate de sodium et on évapore le solvant sous pression réduite. On obtient le composé du titre.

**35f/ Chlorhydrate de 1-[2-(2'-trifluorométhyl-4-biphénylyi)éthyl]-4-(3-trifluorométhylphényl)-1,2,3,6-tétrahydropyridine.**

En opérant comme décrit dans l'exemple 1d mais en utilisant le produit de l'étape précédente au lieu du 2-(4-isobutylphényl)propyl méthanesulfonate, on obtient le composé du titre. P.f. 176-178°C.

EXEMPLE 36

**Oxalate de 1-[2-(3,4-diisobutylphényl)éthyl]-4-(3-trifluorométhylphényl)-1,2,3,6-tétrahydropyridine.**

**36a/** ***1,2-diisobutylbenzène***

A une solution 2M de chlorure d'isopropylmagnesium dans le THF, on ajoute goutte à goutte, sous atmosphère d'azote, une solution de 9,4 g (0,07 mole) de 1,2-diphtalaldéhyde dans 30 ml de THF. On agite ensuite à la température ambiante pendant 2 heures, on verse le mélange dans une solution saturée de chlorure d'ammonium, on évapore le THF sous pression réduite et on extrait à l'éther éthylique. On sèche la phase organique sur du sulfate de sodium et

on évapore le solvant sous pression réduite. On purifie le résidu par chromatographie sur colonne de gel de silice en éluant avec un mélange cyclohexane/acétate d'éthylc = 7/3. On dissout le diol ainsi obtenu dans 110 ml d'éthanol absolu, on y ajoute 5 ml d'acide sulfurique à 96 % et 0,67 g de Pd/C à 10 %. On hydrogène le mélange à pression et température ambiantes. Après consommation de la quantité théorique d'hydrogène (7 heures environ), on filtre le catalyseur, on évapore le solvant, on reprend à l'acétate d'éthyle, on lave avec une solution aqueuse de bicarbonate, ensuite à l'eau, on sèche la phase organique et on évapore le solvant sous pression réduite. On obtient 3,9 g du composé du titre sous forme huileuse.

**36b/ *1-bromo-2-(3,4-diisobutylphényl)éthane***

En opérant comme décrit dans l'exemple 4a mais en utilisant le produit de l'étape précédente au lieu de l'isobutylbenzène, on obtient le composé du titre.

**36c/ *Oxalate de 1-[2-(3,4-diisobutylphényl)éthyl]-4-(3-trifluorométhylphényl)-1,2,3,6-tétrahydropyridine***

En opérant comme décrit dans l'exemple 4b mais en utilisant le produit de l'étape précédente au lieu du 1-bromo-2-(4-isobutylphényl)éthane et en traitant la base ainsi obtenue avec de l'acide oxalique au lieu de l'acide chlorhydrique, on obtient le composé du titre. P.f. 175-178°C.

EXEMPLE 37

**Oxalate de 1-[2-(3,4-dipropylphényl)éthyl]-4-(3-trifluorométhylphényl)-1,2,3,6-tétrahydropyridine.**

**37a/ *1,2-dipropylbenzène***

En opérant comme décrit dans l'exemple 36a mais en utilisant la 1,2-dicarboxyaldéhyde au lieu de la 1,2-diphtalaldéhyde, on obtient le composé du titre.

**37b/ *1-bromo-2-(3,4-dipropylphényl)éthane***

En opérant comme décrit dans l'exemple 36b mais en utilisant le produit de l'étape précédente au lieu du 1,2-diisobutylbenzène, on obtient le composé du titre.

**37c/ Oxalate de 1-[2-(3,4-dipropylphényl)éthyl]-4-(3-trifluorométhylphényl)-1,2,3,6-tétrahydropyridine.**

En opérant comme décrit dans l'exemple 36c mais en utilisant le produit de l'étape précédente au lieu du 1-bromo-2-(3,4-diisopropylphényl)éthane, on obtient le composé du titre. P.f. 180-182°C.

EXEMPLE 38

**1-[2-(4-cyclohexylphényl)éthyl]-4-(6-chloropyrid-2-yl)-1,2,3,6-tétrahydropyridine.**

En opérant comme décrit dans l'exemple 22d mais en utilisant le 1-bromo-2-(4-cyclohexylphényl)éthane (préparé selon l'exemple 19a) au lieu du 4-(2-bromoéthyl)biphényle, on obtient le composé du titre.

EXEMPLE 39

**Chlorhydrate de 1-[2-(4-isobutylphényl)propyl]-4-(6-chloropyrid-2-yl)-1,2,3,6-tétrahydropyridine.**

En opérant comme décrit dans l'exemple 1d mais en utilisant la 4-(6-chloropyrid-2-yl)-1,2,3,6-tétrahydropyridine (préparée selon l'exemple 22c) au lieu de la 4-(3-trifluorométhylphényl)-1,2,3,6-tétrahydropyridine, on obtient le composé du titre. P.f. 185-190°C.

## Revendications

**1.** Procédé de préparation des composés de formule (t)

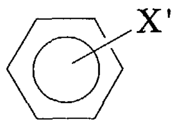

(t)

où le benzène peut être éventuellement substitué et X' est un phényle, éventuellement mono- ou polysubstitué par un halogène, $CF_3$, $(C_1-C_4)$alkyle, $(C_1-C_4)$alcoxy, cyano, amino, mono- ou di-$(C_1-C_4)$alkylamino, $(C_1-C_4)$acylamino, carboxy, $(C_1-C_4)$alcoxycarbonyle, aminocarbonyle, mono- ou di-$(C_1-C_4)$alkylaminocarbonyle, amino$(C_1-C_4)$alkyle, hydroxy$(C_1-C_4)$alkyle ou halogéno$(C_1-C_4)$alkyle, ledit procédé consistant à faire réagir un composé de formule (w)

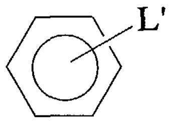

(w)

où le benzène peut être éventuellement substitué et L' représente un groupe partant, avec un acide benzèneboronique de formule X'-$B(OH)_2$ où X' est tel que défini ci-dessus, en présence d'un sel de palladium, d'une base forte et d'un agent de transfert de phase, en milieu aqueux.

**2.** Procédé selon la revendication 1 pour la préparation des composés de formule (t')

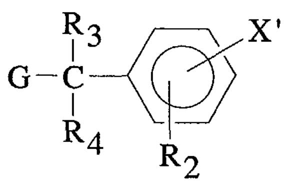

(t')

où $R_2$ représente l'hydrogène, un halogène, un hydroxyle, un groupe $CF_3$, $(C_3-C_4)$alkyle ou $(C_1-C_4)$alcoxyle, $R_3$ et $R_4$ représentent chacun l'hydrogène ou un $(C_1-C_3)$alkyle, X' est tel que défini pour (t) dans la revendication 1 et G représente un groupe carboxy ou L-$CH_2$- où L est un groupe partant, qui consiste à faire réagir un composé de formule (w')

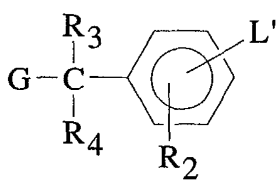

(w')

où $R_2$, $R_3$, $R_4$ et G sont tels que définis ci-dessus et L' est tel que défini pour (w) dans la revendication 1, avec un acide benzèneboronique de formule X'-$B(OH)_2$ où X' est tel que défini ci-dessus, en présence d'un sel de palladium, d'une base forte et d'un agent de transfert de phase, en milieu aqueux.

**3.** Procédé selon la revendication 1 ou 2, **caractérisé en ce que** le groupe partant L' est le brome ou le groupe trifluorométhylsulfonyloxy.

**4.** Procédé selon l'une quelconque des revendications 1 à 3, **caractérisé en ce que** le sel de palladium utilisé est l'acétate de palladium.

**5.** Procédé selon l'une quelconque des revendications 1 à 4, **caractérisé en ce que** la base forte utilisée est choisie parmi les hydroxydes et les carbonates de métaux alcalins.

6. Procédé selon l'une quelconque des revendications 1 à 5, **caractérisé en ce que** l'agent de transfert de phase est un halogénure de tétraalkylammonium.

7. Procédé selon l'une quelconque des revendications 1 à 6, **caractérisé en ce que** la réaction est conduite à une température comprise entre 30°C et le reflux.